# EUROPÄISCHE PATENTSCHRIFT

(11) **EP 2 284 534 B1**
(45) Veröffentlichungstag und Bekanntmachung des Hinweises auf die Patenterteilung: **28.12.2011**
(21) Anmeldenummer: 09010442.3
(22) Anmeldetag: 13.08.2009
(51) Int. Cl.: G01N 33/52

(54) **Testelement zur Analyse einer Körperflüssigkeit**
Test element for analysing a bodily fluid
Elément de test destiné à l'analyse d'un liquide corporel

(43) Veröffentlichungstag der Anmeldung: 16.02.2011
(73) Patentinhaber: Roche Diagnostics GmbH, 68305 Mannheim (DE); F.Hoffmann-La Roche AG, 4070 Basel (CH)
(72) Erfinder: Haar, Hans-Peter, 69168 Wiesloch (DE)
(74) Vertreter: Durm & Partner

(56) Entgegenhaltungen:
- EP-A2- 1 155 742
- WO-A2-2008/051271
- US-A- 5 399 315
- US-A1- 2003 148 401
- US-A1- 2006 246 416

## Beschreibung

Die Erfindung betrifft ein Testelement zur Analyse einer Körperflüssigkeit auf einen darin enthaltenen Analyten sowie ein Verfahren zu seiner Herstellung und ein Analysesystern, zu dem ein erfindungsgemäßes Testelement und ein zu dessen Auswertung geeignetes, speziell angepasstes Auswertegerät gehören. Derartige Testelemente und Analysesysteme werden vor allem für medizinische Analysen verwendet. Ein besonders wichtiges Anwendungsgebiet der Erfindung ist die Untersuchung sehr kleiner Probenmengen (vorzugsweise weniger als 1 µl, besonders bevorzugt weniger als 0,5 µl), die typischerweise durch einen Stich in die Haut gewonnen werden.

Die Analyse erfolgt mittels eines Reagenzsystems, das in der Regel aus mehreren Reagenzien und Hilfsstoffen besteht, die in das Testelement integriert sind und deren Reaktion mit einem in der Körperflüssigkeit enthaltenen Analyten zu einer Änderung einer an dem Testelement messbaren, für das gewünschte analytische Ergebnis charakteristischen Messgröße führt. Die Erfindung richtet sich speziell auf sogenannte optische Analyseelemente und - systeme, bei denen die für die Analyse charakteristische Änderung des Testelementes optisch messbar ist. In der Regel führt bei optischen Testelementen die Reaktion zur Änderung der Farbe einer Schicht, die Bestandteil des Testelementes ist und als Detektionsschicht bezeichnet wird. Die Farbänderung der Detektionsschicht wird photometrisch vermessen. Neben diesen colorimetrischen Testsystemen sind auch andere optisch auswertbare Testsysteme bekannt, beispielsweise Systeme, bei denen die Messgröße ein Fluoreszenzsignal ist. Die Erfindung bezieht sich allgemein auf Analysesysteme, bei denen die Analyse auf der Messung einer an dem Testelement optisch messbaren Messgröße besteht, wobei das Messergebnis für das gewünschte analytische Resultat charakteristisch ist. Nachfolgend wird ohne Beschränkung der Allgemeinheit auf Farbänderungen als Beispiel für optisch messbare Messgrößen Bezug genommen.

Analysesysteme sind in zahlreichen Varianten zur quantitativen und qualitativen Bestimmung unterschiedlicher Analyten gebräuchlich. Besonders große medizinische und wirtschaftliche Bedeutung haben Systeme zur Bestimmung der Glucosekonzentration im Blut von Diabetikern. Die Erfindung ist insbesondere für solche Systeme geeignet. Sie ist jedoch nicht darauf beschränkt. Ein anderer wichtiger Analyt ist beispielsweise Cholesterin.

Insbesondere richtet sich die Erfindung auf Anwendungsfälle, bei denen die Analyse durch den Patienten selbst durchgeführt wird, um seinen Gesundheitszustand zu überwachen ("home-monitoring"). Dabei kommt es besonders auf eine einfache Handhabung an. Außerdem müssen die Auswertegeräte möglichst klein, leicht und robust sein.

Optische Testelemente haben eine Tragstruktur, die in der Regel aus Kunststoff besteht und meist als schmaler, länglicher Kunststoffstreifen ausgebildet ist ("Teststreifen"). Ein Teilbereich des Testelementes, in welchem mindestens ein Teil der Reagenzien und die Detektionsschicht, in der die Farbbildung stattfindet, lokalisiert ist, wird als Testfeld (englisch: "chemistry pad") bezeichnet.

Das Testfeld kann aus einer oder mehreren Schichten bestehen, die in Fluidkontakt zueinander stehen und in der Regel parallel zueinander verlaufen. Reagenzienhaltige Schichten ("Reagenzschichten") des Testfeldes bestehen oft aus einem saugfähigen porösen Schichtmaterial (beispielsweise Papier, Vlies oder eine Kunststoffmembran) mit imprägnierten Reagenzien.

Daneben gibt es schon seit langem Testfelder, bei denen mindestens eine Reagenzschicht auf ein geeignetes Trägermaterial durch Beschichten aufgebracht ist ("coated test layers"). Zur Herstellung einer solchen Schicht, die auch als "Reagenzfilm" bezeichnet wird, werden die Reagenzien mit einem Binde- oder Verdickungsmittel gemischt, um eine zähflüssige Beschichtungsmasse zu bilden. Nach dem Beschichten und Trocknen wird ein dünner Film auf dem Trägermaterial gebildet. Das Verdickungs- oder Bindemittel wird deshalb auch als "Filmbildner" bezeichnet. Der Reagenzfilm muss so ausgebildet sein, dass bei Kontakt mit der wässrigen Probenflüssigkeit die für die Analyse erforderliche Reaktion mit den darin enthaltenen Reagenzien stattfindet. Um dies zu gewährleisten ist der Reagenzfilm in der Regel zumindest teilweise löslich und/oder quellfähig. Er kann auch als "Öffner" geeignete Partikel enthalten, die das Eindringen der Probenflüssigkeit erleichtern. Ein charakteristisches Merkmal eines Reagenzfilmes in diesem Sinne besteht darin, dass er, im Gegensatz zu den erwähnten Reagenzschichten auf Basis eines saugfähigen porösen Schichtmaterials, keine auch nach dem Einbringen der wässrigen Probenflüssigkeit dauerhaft feste poröse Tragstruktur hat.

Für die Erfindung sind insbesondere enzymatische Reagenzsysteme geeignet. Sie enthalten ein Enzym, das mit dem Analyten spezifisch reagiert. Im Falle der Glucoseanalyse wird als Enzym beispielsweise Glucosedehydrogenase (GDH) verwendet, wobei im Rahmen der Erfindung besonders bevorzugt PoGI-abhängige GDH eingesetzt wird, welche auch als GlucDOR (glucose dye oxidoreductase) bezeichnet wird. Die Reaktion zwischen Glucose und Enzym führt zu einer weiteren Reaktion einer Indikatorsubstanz, die mit einer Farbänderung verbunden ist. Meist enthält das Reagenzsystem weitere Reaktionsbestandteile, insbesondere einen Mediator, der den mit den Reaktionen verbundenen Elektronentransfer von dem Enzym auf den Indikator erleichtert und dadurch eine schnellere Analyse ermöglicht.

Nähere Einzelheiten über Testelemente und zugehörige Analysesysteme können der einschlägigen Literatur entnommen werden. Eine Übersicht gibt beispielsweise der Artikel von J. Hönes et al "The Technology Behind Glucose Meters: Test Strips", in Diabetes Technology & Therapeutics, 2008, Supplement 1, pp S 10 bis S 26.

Die Präzision der analytischen Resultate ist bei optischen Analysesystemen wesentlich von der Homogenität der Farbbildung in der Detektionsschicht des Testfeldes abhängig. Um Inhomogenitäten, die beispielsweise durch Chromatographie-Effekte beim Eindringen der Probenflüssigkeit entstehen können, zu reduzieren, ist es gebräuchlich, eine zu dem Testfeld benachbarte Spreitschicht vorzusehen, deren dem Testfeld zugewandte Flüssigkeitsaustrittsseite mit einer Flüssigkeitseintrittsseite des Testfeldes in Fluidkontakt steht. Die Spreitschicht ist so ausgebildet, dass eine in sie eindringende Flüssigkeit auf ihrer Flüssigkeitsaustrittsseite gleichmäßig verteilt wird. Um dies zu erreichen, werden für die Spreitschicht Materialien verwendet, in denen aufgrund von Kapillaraktivität eine rasche Ausbreitung der wässrigen Probenflüssigkeit über die ganze Fläche der Schicht stattfindet. Geeignet sind beispielsweise relativ lockere Faserverbundstrukturen, insbesondere Gewebe, Gewirke oder Vliese, aus hydrophilen (oder hydrophilisierten) Fäden oder Fasern.

Die Spreitschicht kann sowohl ein Bestandteil des Testfeldes als auch ein Bestandteil eines dem Testfeld benachbarten anderen Bauteils des Testelementes sein. Beispielsweise wird in der EP 1 360 931 A1 ein Testelement beschrieben, dessen Tragstruktur von einem Bauteil gebildet wird, das eine scharfe Spitze zum Einstechen in die Haut hat und als Stechelement bezeichnet wird. Von der Spitze des Stechelementes führt ein Kapillarkanal in dessen hinteren Teil, in dem sich bei einer der dargestellten Ausführungsformen ein für eine optische Auswertung geeignetes Testfeld befindet. Das Stechelement hat dabei eine Vielzahl von Seitenkanälen, die von dem Hauptkanal, der die Verbindung zu der Spitze herstellt, rechtwinklig abzweigen und die Probenflüssigkeit über die gesamte Fläche des Testfeldes verteilen. Diese Kanäle bilden eine zu dem Testfeld benachbarte Spreitschicht, welche nicht Bestandteil des Testfeldes selbst, sondern Bestandteil eines benachbarten Bauteils, hier des Stechelementes, ist.

Der Erfindung liegt das technische Problem zugrunde, ein verbessertes Testelement zur Verfügung zu stellen. Es soll eine sehr gute Messgenauigkeit insbesondere bei einem optischen Test gewährleisten, insbesondere bei sehr kleinen Probenmengen. Für die Analyse stehen nur sehr kleine Probenmengen zur Verfügung, wenn ein möglichst schmerzarmer Einstich gewährleisten werden soll. Dieser bedingt eine geringe Stichtiefe und eine kurze Verweildauer des Stechelements in der Haut.

Dieses technische Problem wird bei einem Testelement der vorstehend geschilderten Art durch eine Kombination folgender Maßnahmen gelöst:
- Die Spreitschicht hat eine flächige Matrixstruktur mit einer Mehrzahl von Zellen, die je einen Zellhohlraum aufweisen, wobei die Zellhohlräume der Zellen durch Passagen in offener Verbindung zueinander stehen und somit eine Fluidverbindung ermöglichen;
- die Zellen sind in einer Ebene angeordnet;
- die Spreitschicht ist aus einer Folie mittels eines sich anschließenden Schichtprofilierungsverfahrens hergestellt.

Im Gegensatz zu der erwähnten EP 1 360 931 A1 wird im Rahmen der vorliegenden Erfindung also keine Kanal-Spreitstruktur, sondern eine Zellmatrix-Spreitstruktur verwendet. Die Zellen sind als Matrix in einer Ebene angeordnet. Die Raumrichtungen, in der sich die Ebene erstreckt, werden mit X und Y bezeichnet. "Matrixstruktur" bedeutet, dass sowohl in X-Richtung als auch in Y-Richtung mehrere Zellen (vorzugsweise in mindestens einer Richtung mindestens vier Zellen) benachbart sind. Nähere Erläuterungen über bevorzugte Anordnungen und Gestaltungen der Zellen werden weiter unten gegeben. Beispielsweise ist die Dimension (Abmessung) der Zellen in einer Richtung der X-Y-Ebene bevorzugt höchstens doppelt, besonders bevorzugt höchstens 1,5 mal so groß wie die andere Dimension in der X-Y-Ebenenrichtung.

Die Spreitschicht wird vorzugsweise aus einer Kunststofffolie hergestellt, wobei für manche Anwendungszwecke auch eine Metallfolie oder eine Verbundfolie verschiedener Materialien geeignet ist. Als "Schichtprofilierungsverfahren" wird ein Verfahren bezeichnet, durch das auf (mindestens) einer Seite der Folie ein für die Spreitschichtfunktion geeignetes dauerhaftes Flächen-Profil (Relief) erzeugt wird. Vorzugsweise wird ein Prägeverfahren eingesetzt, das weiter unten noch näher erläutert wird. Grundsätzlich sind aber auch andere Schichtprofilierungsverfahren geeignet, wie beispielsweise die in der Kunststofftechnik weitgehend gebräuchlichen Tiefziehverfahren oder auch Verfahren, bei denen die gewünschte Formgebung - zumindest teilweise - mittels lokaler Erwärmung, beispielsweise mittels eines Lasers, erreicht bzw. unterstützt wird. Eine Profilerzeugung oder -änderung kann neben Lasern auch durch Ätzen bzw. Ätztechniken erzielt werden.

Die Erfindung kombiniert somit in neuartiger Weise technische Elemente aus zwei bisher getrennten Entwicklungslinien der Spreitschichtentwicklung:
- Einerseits handelt es sich um eine Zellmatrix-Spreitschicht, insoweit übereinstimmend mit den vielfach gebräuchlichen Faserverbundstrukturen, insbesondere Spreitnetzen aus gewebten oder gewirkten Fäden.
- Andererseits bildet die profilierte Folie der Spreitschicht ein einziges (einstückiges) Bauteil, insoweit übereinstimmend mit der Spreitstruktur des Stechelementes der EP 1 360 931 A1, aber abweichend von den bisher gebräuchlichen Faserverbundstrukturen.

Im Vergleich zu einer Kanalstruktur wird mit einer Zellmatrixstruktur eine gleichmäßigere Verteilung der Flüssigkeit und damit bessere Homogenität der Farbbildung und erhöhte Messgenauigkeit erreicht. Außerdem ist das Flüssigkeitsvolumen der Spreitschicht geringer, so dass mit sehr kleinen Probenmengen gearbeitet werden kann.

Im Rahmen der Erfindung wurde erkannt, dass die bei größeren Probenmengen sehr gut funktionierenden Spreitnetze bei sehr kleinen Probenvolumina von weniger als 1 µl, insbesondere weniger als 300 nl oder weniger als 100 nl, nachteilig sind. Es wurde festgestellt, dass optische Tests, insbesondere soweit sie mit einer durch Beschichten aufgebrachten Testschicht arbeiten, Präzisionsprobleme haben, wenn die Dicke der von der Konstruktion der Spreitschicht bestimmten, der Eintrittsseite des Testfelds benachbarten Flüssigkeitsschicht zu gering ist.

Im Rahmen der Erfindung wurde festgestellt, dass es trotz des Bestrebens nach einem möglichst kleinen Probenvolumen vorteilhaft ist, das Testelement so zu konstruieren, dass die Flüssigkeitseintrittsseite des Testfeldes während der Reaktion mit einer Flüssigkeitsschicht in Kontakt steht, deren Schichtdicke in Abhängigkeit von der Messzeit einen Mindestwert hat. Dabei kommt es auf die Dimension der Flüssigkeitsschicht senkrecht zu der Testfeldebene an. Diese Abmessung wird nachfolgend auch als Flüssigkeitssäule bezeichnet.

Die erforderliche Mindesthöhe der Flüssigkeitssäule (d.h. Mindestdicke der Flüssigkeitsschicht) hängt von der Messzeit ab, für die das Testelement ausgebildet ist. Insbesondere bei optischen Messungen gilt: Je kürzer die Messzeit, d.h. die Zeit zwischen der ersten Kontaktierung des Testfeldes mit der Probenflüssigkeit und der optischen Messung (im Falle mehrerer optischer Messungen der letzten optischen Messung), ist, desto kleiner ist die für die eine gute Messgenauigkeit notwendige Mindesthöhe der Flüssigkeitssäule. Zu dieser Korrelation können folgende Zahlenangaben gemacht werden:
- Wenn das Testelement für eine Messzeit von maximal 15 Sekunden ausgebildet ist, sollte die Kapillarstruktur so ausgebildet sein, dass die Schichtdicke der an die Flüssigkeitseintrittsseite des Testfeldes angrenzenden durch die Kapillarstruktur bestimmten Flüssigkeitsschicht mindestens 100 µm beträgt.
- Wenn das Testelement für eine Messzeit von maximal 5 Sekunden ausgebildet ist, sollte die Kapillarstruktur so ausgebildet sein, dass die Schichtdicke der an die Flüssigkeitseintrittsseite des Testfeldes angrenzenden durch die Kapillarstruktur bestimmten Flüssigkeitsschicht mindestens 50 µm beträgt.
- Wenn das Testelement für eine Messzeit von maximal 1 Sekunde ausgebildet ist, sollte die Kapillarstruktur so ausgebildet sein, dass die Schichtdicke der an die Flüssigkeitseintrittsseite des Testfeldes angrenzenden durch die Kapillarstruktur bestimmten Flüssigkeitsschicht mindestens 20 µm beträgt.

Diese Flüssigkeitsschichtdicke sollte deswegen mindestens 20 µm betragen. Um mittels einer Faserverbundstruktur eine solche Flüssigkeitsschichtdicke zu gewährleisten, müssen deren Fasern einen entsprechend großen Durchmesser haben. Dies führt zu Inhomogenitäten der Flüssigkeitsverteilung in der Nachbarschaft der Eintrittsseite des Testfeldes und daraus resultierend Inhomogenitäten der Farbbildung und Messungenauigkeiten. Zudem ist die Fließgeschwindigkeit einer Flüssigkeit durch eine Faserverbundstruktur, z.B. ein Vlies, zu gering, was zu einer nicht vernachlässigbaren Änderung eines Messergebnisses führt.

Im Rahmen der Erfindung wird dieses Problem dadurch überwunden, dass die Spreitschicht (engl. spreading layer) mittels des Schichtprofilierungsverfahrens hergestellt wird und für den jeweiligen Anwendungsfall optimiert werden kann. Das Profil der Folie kann relativ frei den Erfordernissen angepasst werden. Die Herstellung aus einer Folie mittels Schichtprofilierungsverfahren ist preiswert und garantiert eine konstante Schichthöhe mit hoher Genauigkeit auch bei kleinen Spreitschichthöhen von höchstens 60 µm, sogar bei Schichthöhen von höchstens 40 µm oder 20 µm.

Beispielsweise geht bei einer Messzeit von 5 Sekunden die Dicke der Flüssigkeitsschicht von weniger als ca. 50 µm in das Messergebnis ein und kann nicht vernachlässigt werden. Trotzdem kann mit Spreitschichten, die mit dem Schichtprofilierungsverfahren hergestellt sind, eine Messung auch bei kleineren Schichtdicken erfolgen. Dazu ist es wichtig, eine konstante Schichtdicke der Spreitschicht zu gewährleisten, die nicht nur innerhalb einer einzelnen Spreitschicht konstant sein muss, sondern auch innerhalb einer Produktreihe bei einer Massenfertigung und möglichst über verschiedene Produktionschargen hinweg. Durch das Schichtprofilierungsverfahren und insbesondere durch das erfindungsgemäße Herstellungsverfahren nach Anspruch 18, das noch näher beschrieben wird, werden diese Anforderungen erfüllt. Eine sehr konstante Spreitschichtdicke kann auch bei einer Massenproduktion zuverlässig gewährleistet werden.

Insoweit hat sich die Erfindung auch als überlegen gegenüber Spreitschichten erwiesen, die als Kunststoffmembran, also als schwammartig poröse Kunststoffschicht, ausgebildet sind. Diese gewährleisten durch ihre Zellmatrixstruktur zwar eine gleichmäßige Verteilung der Flüssigkeit, jedoch ist der erforderliche Zeitbedarf, um die Struktur mit Flüssigkeit zu füllen relativ hoch und erhöht damit die Zeit zum Durchführen eines Tests.

Gemäß einer bevorzugten Ausführungsform ist die Dimension (Abmessung) der Zellen senkrecht zu der X-Y-Ebene (also in "2-Richtung") verschieden von den Dimensionen der Zellen in Richtung der Ebene (also in X- und Y-Richtung). Bevorzugt sind die Dimensionen in X-Richtung und in Y-Richtung ebenfalls unterschiedlich. Die Zellen können also rechteckig oder quadratisch sein. Selbstverständlich können die Zellen in X-Y-Ebene auch rund oder elliptisch ausgebildet sein. Da die Dimension in Z-Richtung (Höhe der Zellen bzw. Dicke der Spreitschicht) unabhängig von den beiden anderen Dimensionen ist, kann die Zelle beispielsweise die Form eines Zylinders oder eines Quaders aufweisen. Auf diese Weise lassen sich Zellen gestalten, die eine Höhe (Dimension in Z-Richtung) von z. B. 50 µm haben, jedoch kleinere Dimensionen in der X-Y-Ebene. Mit einer derartigen Spreitschicht können in der Praxis Flüssigkeitsvolumen von höchstens 150 nl oder von höchstens 50 nl und darunter vermessen werden. Das dabei von der Spreitschicht aufzunehmenden Volumen ist bevorzugt höchsten 10 nl, besonders bevorzugt höchstens 6 nl oder höchstens 5 nl groß. Die "restliche" Flüssigkeitsmenge (Differenz zu 100 nl oder 50 nl) wird von dem Transferkanal bei der Zuführung der Flüssigkeit aufgenommen ("verbraucht"). Die Spreitschichthöhe begrenzt die Dicke der Flüssigkeitsschicht innerhalb der Spreitschicht. Bevorzugt ist die Dicke der Flüssigkeitsschicht höchstens 150 µm.

In einer bevorzugten Ausführungsform ist die Dimension der Zellen in Z-Richtung, also senkrecht zu der X-Y-Ebene, größer als die kleinere der beiden Dimensionen in Richtung der Ebene. Es hat sich als vorteilhaft erwiesen, wenn die Ausdehnung der Zelle in Z-Richtung wenigstens 1,5 mal so groß ist wie die Ausdehnung der kleineren Dimension in Richtung der Ebene. Besonders bevorzugt ist die Dimension in Z-Richtung wenigstens doppelt so groß wie die kleinere Dimension in Richtung der Ebene.

Um die jeweiligen Zellen der Spreitschicht mit Flüssigkeit zu füllen, hat die Spreitschicht eine kapillaraktive Matrixstruktur mit einer Mehrzahl von Zellen, die auch als Kapillarstruktur bezeichnet wird und für die Spreitung bzw. Verteilung der Flüssigkeit sorgt. Im Sinne der Erfindung wird unter dem Begriff Spreitung die Verteilung einer Flüssigkeit derart verstanden, dass eine vordefinierte (Mindest-)Flüssigkeitsdicke in der Spreitschicht gebildet wird. Die Flüssigkeit wird dabei (bevorzugt gleichmäßig) auf eine Mehrzahl von benachbarten Zellen verteilt, in denen sich dieselbe Flüssigkeitshöhe in den Zellen einstellt. Bevorzugt sind alle Zellen der Spreitschicht mit derselben Flüssigkeitshöhe gefüllt. Für viele Anwendungen hat sich eine Dicke der Flüssigkeitsschicht von wenigstens 30 µm als positiv erwiesen. Die Flüssigkeitsschicht in der Spreitschicht ist bevorzugt höchstens 100 µm groß, bevorzugt höchstens 70 µm. In einigen Anwendungsfällen kann die Schichtdicke höchstens 50 µm oder 40 µm betragen.

Im Rahmen der Erfindung wurde erkannt, dass bei der Analyse von kleinen und kleinsten Blutmengen von höchstens 100 nl oder höchstens 50 nl, Nachteile bei der Verwendung von herkömmlichen Spreitstrukturen bestehen, wenn gleichzeitig eine Mindestflüssigkeitsschichthöhe von wenigstens 40 µm oder wenigstens 50 µm gefordert ist. Bei gewebten Spreitstrukturen aus Netzfäden beträgt dazu die Fadendicke wenigstens 40 µm. Um ein kleines Flüssigkeitsvolumen auf mehrere Zellen zu verteilen (wenigstens 20 bis 25 Zellen), muss das Volumen der Zellen entsprechend klein sein. Die Gesamtfläche der Zellöffnungen an der Flüssigkeitsaustrittsseite ist dann kleiner als 50 % der Gesamtfläche der Flüssigkeitsaustrittsseite. Die Spreitschicht weist keine optische Homogenität auf. Eine zuverlässige, aussagekräftige Messung ist damit unmöglich, da der Einfluss der Netzfäden nicht mehr vernachlässigbar wäre. Dieses Problem wird durch eine (bevorzugt einstückige) Spreitschicht überwunden, insbesondere durch eine Spreitschicht mit asymmetrischen Zellen, wie sie im Rahmen der Erfindung entwickelt wurde. Die erfindungsgemäße (einstückige) Spreitschicht hat eine Zellmatrix-Spreitstruktur mit im Vergleich zu den Zellhohlräumen geringen Zellwandstärken, wobei die Dicke der Zellwände unabhängig von der Dicke (Z-Richtung) der Spreitschicht ist.

Im Rahmen der Erfindung wurde festgestellt, dass dieses Problem sowie das der Erfindung zugrunde liegende technische Problem auch mit einem Testelement mit den Merkmalen des Anspruchs 13 gelöst werden kann. Ein derartiges Testelement hat ein Testfeld und eine Spreitschicht. Die Spreitschicht ist als flächige Matrixstruktur mit einer Mehrzahl von Zellen ausgebildet, die in einer Ebene angeordnet sind. Die Zellen weisen Zellhohlräume auf, die durch Flüssigkeitspassagen in Fluidverbindung miteinander stehen. Die erfindungsgemäße Spreitschicht ist so ausgebildet, dass mindestens eine der Dimensionen der Zellen in der Ebene höchstens 40 µm beträgt, während die Dimension der Zellen senkrecht zu der Ebene mindestens 35 µm beträgt. Unter Dimension wird hierbei der Abstand in X- bzw. Y-Richtung zwischen zwei gegenüberliegenden Zellwänden verstanden. Dabei muss die Dimension der Zelle senkrecht zu der Ebene größer sein als die kleinere der Dimensionen der Zellen in Richtung der Ebene. Die Spreitschicht ist also so ausgebildet, dass die Zellen eine größere Höhe aufweisen als ihre kleinste Abmessung in X-Y-Ebene. Bevorzugt ist die Dimension der Zellen senkrecht zur Ebene (Höhe der Zellen) wenigstens 1,5 x, besonders bevorzugt wenigstens 2 x so groß wie die kleineren Dimensionen in der Ebene.

Typischerweise hat die Zelle einen quadratischen Querschnitt mit einer Dimension in X-Y-Ebene von 40 µm. Bei Zellen mit einem quadratischen oder runden Querschnitt in Ebenenrichtung hat die Zelle die Form eines senkrecht stehenden Quaders bzw. Zylinders. Auf diese Weise lassen sich Zellen mit einer kleinen Querschnittsfläche erzeugen, die jedoch die erforderliche und gewünschte Mindestflüssigkeitsschichtdicke zur Verfügung stellen können. Bevorzugt ist die Dimension in Z-Richtung wenigstens 50 µm, besonders bevorzugt 60 µm groß.

Eine Flüssigkeitsschichtdicke von bevorzugt wenigstens 60 µm gewährleistet, dass während der typischerweise zwischen 5 und 7 Sekunden dauernden Reaktionszeit der Probenflüssigkeit mit dem Testfeld Diffusionsvorgänge von Glucose und weiteren Reaktionsteilnehmern stattfinden können, die nicht von der Schichtdicke der Flüssigkeit begrenzt wird. Auf diese Weise wird sichergestellt, dass die ablaufende Reaktion von der aufgegebenen Flüssigkeitsmenge unabhängig ist. In das Messergebnis geht lediglich die Flächenkonzentration der Reaktionsteilnehmer ein und nicht das zur Verfügung gestellte Flüssigkeitsvolumen. Mit dem erfindungsgemäßen Testelement wird diese Voraussetzung für kleinste Flüssigkeitsvolumen von maximal 100 nl, bevorzugt maximal 50 nl, erfüllt.

Es wurde festgestellt, dass eine Spreitschicht mit derart dimensionierten Zellen für die Messung von kleinen und kleinsten Flüssigkeitsvolumen geeignet ist. Dabei spielt es keine Rolle, auf welche Weise die Spreitstruktur hergestellt ist. Sie muss nicht mit einem Schichtprofilierungsverfahren beispielsweise aus einer Folie erzeugt werden, wenngleich dies möglich und bevorzugt ist. Auch eine Spreitschicht mit derartigen Zellen, die beispielsweise aus einer Gewebestruktur hergestellt sein könnte, ist zur Lösung dieses Problems durchaus geeignet.

Als weitere Anforderung besteht bei den Testelementen, dass die Flüssigkeit in der Spreitschicht sehr schnell transportiert und auf eine Vielzahl von Zellen verteilt werden muss und sich die gewünschte Flüssigkeitsschichtdicke an die Reaktionsschicht des Testfeldes angrenzend einstellt. Zwar breitet sich die Flüssigkeit in der Reaktionsschicht des Testfeldes relativ langsam aus. Aufgrund der geringen Dichte der Reaktionsschicht von in der Regel 20 µm und darunter wird die Reaktionsschicht jedoch sehr schnell mit Flüssigkeit gefüllt. Da die Form der Zellen an die jeweiligen Bedürfnisse angepasst werden kann und die Zellen durch Flüssigkeitspasssagen miteinander verbunden sind, erfüllt die erfindungsgemäße Spreitschicht diese Forderung auch bei einer geringen Dicke der Spreitschicht und bei kleinen Flüssigkeitsmengen. Unterstützt wird die schnelle und zügige Verteilung durch die in den Zellen auftretende Kapillarwirkung.

Um bei rechteckförmigen Spreitschichten eine gleichmäßige Verteilung zu gewährleisten, können die einzelnen Zellen der matrixförmigen Spreitschicht angepasst sein. Um ein Voranschreiten in einer Richtung der Ebene (z. B. Y-Richtung) schneller erfolgen zu lassen als in der anderen Richtung der Ebene, können die einzelnen Zellen ebenfalls rechteckförmig ausgebildet sein. Durch die flexible Gestaltung der bevorzugt einstückigen und im Schichtprofilierungsverfahren hergestellten Spreitschicht kann eine nahezu zeitgleiche Verteilung der Flüssigkeit in der Ebene (an der Flüssigkeitsaustrittsseite) erzielt werden. Eine entsprechende Anpassung der Flüssigkeitspassagen kann diesen Effekt unterstützen.

Bevorzugt ist also ein gleichzeitiges Füllen der Zellen auch bei Zellen möglich, die von der Aufgabenstelle weiter entfernt sind. Als gleichzeitig wird in diesem Sinne eine Verteilung verstanden, bei der die betreffenden Zellen der Spreitschicht innerhalb einer definierten Zeitspanne mit der Mindestflüssigkeitsschichtdicke gefüllt sind, so dass die gewünschte Messgenauigkeit erzielt wird und die analytischen Ergebnisse nicht unakzeptabel voneinander abweichen. Eine definierte Zeitspanne von weniger als 100 ms wird bei einer Reaktionszeit für die Messung bzw. einer Messzeit von ca. 3 bis 5 Sekunden als gleichzeitig angesehen. Bei Reaktionszeiten von 1 bis 3 Sekunden wird eine Zeitspanne von weniger als ca. 50 ms als gleichzeitig angesehen, bei längeren Reaktionszeiten (30 bis 60 Sekunden) etwa 500 ms bis 1.000 ms.

Ein bevorzugtes Testelement hat ein Testfeld mit mindestens einem Teil der Reagenzien und eine parallel verlaufende Spreitschicht, deren Flüssigkeitsaustrittsseite in Fluidkontakt zu einer Flüssigkeitseintrittsseite des Testfelds steht. Die Spreitschicht hat eine flächige Matrixstruktur mit einer Mehrzahl von Zellen in einer Ebene, die durch Fluidpassagen in Fluidverbindung stehen und deren Höhe (Richtung senkrecht zur Ebene) wenigstens 25 µm oder 30 µm und höchstens 70 µm oder 50 µm ist und deren Dimensionen in Richtung der Ebene wenigstens 20 µm und höchstens 50 µm sind. Die Zellen sind bevorzugt asymmetrisch. Die Spreitschicht hat ein Aufnahmevolumen von höchstens 15 nl, bevorzugt höchstens 10 nl, besonders bevorzugt höchstens 5 nl, bei einer Fläche von höchstens 0,5 mm² (0,7 mm x 0,7 mm), bevorzugt höchstens 0,25 mm² (0,5mm x 0,5 mm). Die Größe der Spreitschicht in Richtung der Ebene ist wenigstens 0,2 mm, bevorzugt wenigstens 0,4 mm und höchstens 1 mm, bevorzugt höchstens 0,7 mm bzw. 0,5 mm.

Im Rahmen der Erfindung wurde erkannt, dass ein Testelement mit einer Spreitschicht, die eine flächige Matrixstruktur mit einer Mehrzahl von Zellen hat und deren Zellen in einer Ebene angeordnet sind, sehr flexibel strukturiert sein kann. Damit ist für verschiedene Anwendungsgebiete eine gute und einfache Anpassung möglich. Ausgehend von diesem Gesichtspunkt wurde erkannt, dass ein Testelement mit den Merkmalen des Anspruchs 15 gegenüber den bekannten Spreitstrukturen und Flüssigkeitshaltestrukturen bei Testelementen deutlich verbessert ist. Ein derartiges Testelement hat ein Testfeld und eine Spreitschicht, die als flächige Matrixstruktur mit einer Mehrzahl von in einer Ebene angeordneten Zellen aufweist. Die Spreitschicht hat in der Ebene wenigstens einen ersten Bereich mit Zellen und wenigstens einen zweiten Bereich mit Zellen, wobei die Größe der Zellen in dem ersten Bereich verschieden ist von der Größe der Zellen in dem zweiten Bereich.

Beispielsweise lässt sich also eine Spreitschicht bilden, deren Randbereiche anders ausgeformt sind als beispielsweise die mittleren Bereiche. Hierdurch ist es zum einen möglich, in einem Bereich aufgebrachte Flüssigkeit mit unterschiedlichen Geschwindigkeiten auf die anderen Bereiche zu verteilen. Zum anderen ergibt sich durch die unterschiedliche Dimension und Größe der Zellen in X-Y-Ebene auch eine örtlich unterschiedliche Steifigkeit. Beispielsweise kann in einzelnen Bereichen der Spreitstruktur die Dicke der Struktur unterschiedlich sein.

Im Rahmen der Erfindung wurde festgestellt, dass eine derartig ausgebildete Spreitschicht mit wenigstens zwei Bereichen mit unterschiedlich großen Zellen nicht nur für die Verteilung von kleinen Flüssigkeitsmengen geeignet ist. Auch größere Flüssigkeitsmengen von mehr als 100 nl, bevorzugt mehr als 300 nl, besonders bevorzugt mehr als 500 nl lassen sich mit einer derartig geformten Spreitschicht ebenfalls gleichmäßig verteilen. Flüssigkeitsmengen von mehr als 1 µl können von einer solchen Spreitschicht ebenfalls gleichmäßig aufgenommen und verteilt werden. Bei größeren Flüssigkeitsmengen können folglich auch die Zellen deutlich größere Querschnitte und Dimensionen aufweisen. Darüber hinaus kann auch die Dicke der Spreitschicht (Höhe der Spreitschicht senkrecht zur X-Y-Ebene) deutlich über 20 µm, bevorzugt über 50 µm, besonders bevorzugt über 150 µm sein.

In einer bevorzugten Ausführungsform ist dabei die Querschnittsfläche in X-Y-Ebenen der Zellen in dem ersten Bereich verschieden von der Querschnittsfläche der X-Y-Ebene in dem zweiten Bereich. In der Spreitschicht können selbstverständlich auch mehrere Bereiche mit Zellen unterschiedlicher Größe angeordnet sein. Vorzugsweise findet dabei eine Gruppierung der Zellen derart statt, dass Zellen gleicher Größe benachbart sind, vorzugsweise also eine Zelle wenigsten eine weitere Zelle gleicher Größe als Nachbarn hat.

Durch die verschieden großen Zellen in bestimmten Bereichen der Spreitschicht und die sich daraus ergebende unterschiedliche Steifigkeit in den Bereichen lässt sich die Spreitschicht krümmen und biegen. Derartige Spreitschichten eignen sich beispielsweise, um auf einem Band aufgewickelt zu werden, wie bei so genannten Tapekassetten. Hierbei ist das Testfeld auf einem (transparenten) bandförmigen Film aufgebracht. Die Spreitschichten werden an dem (Kunststoff-) Film befestigt, beispielsweise angeklebt. Für die Auswertung und Analyse der aufgenommenen Flüssigkeiten ist beispielsweise bekannt, das Band über eine Kante mit einem geringen Radius zu ziehen. Hierbei kommt es im Stand der Technik häufig zu Ablösungen der Spreitschicht, die durch das erfindungsgemäße Testelement mit einer angepassten Steifigkeit vermieden werden, da es sich durch die asymmetrische Struktur der Zellen deutlich leichter krümmen und biegen lässt. Teilweise finden im Stand der Technik auch Ablösungen einer Spreitschicht oder Flüssigkeitshalteschicht von dem Testfeld beim Aufwickeln von bandförmigen Testelementen auf einer Tapekassette statt, da die Wickelradien zum Teil sehr klein sind. Auch dieses Problem wird durch das erfindungsgemäße Testelement gelöst, da die Spreitschicht eine hohe Flexibilität aufweist.

Im Rahmen der Erfindung wurde festgestellt, dass die Herstellung der oben beschriebenen Spreitschicht mit einer Mehrzahl von Zellen zur homogenen Verteilung einer Flüssigkeit mit dem erfindungsgemäßen Verfahren gemäß Anspruch 18 hergestellt werden kann. Dieses Prägeverfahren ist nur ein Beispiel für ein Schichtprofilierungsverfahren. Selbstverständlich können auch andere, dem Fachmann bekannte formgebende Verfahren verwendet werden.

Das erfindungsgemäße Verfahren umfasst mehrere Schritte und wird als Mikroprägeverfahren bezeichnet. In einem ersten Schritt wird eine Folie (oder ein Substrat) bereitgestellt, die bevorzugt aus einem Kunststoff, beispielsweise aus einem Polycarbonat, besteht. Vorteilhafterweise hat die Folie eine definierte Dicke von höchstens 100 µm; bevorzugt von ca. 50 µm. Die Kunststofffolie kann in einem späteren Verfahrensschritt metallisiert oder hydrophilisiert werden, insbesondere wenn die Kunststofffolie sonst keine Nachbehandlung erfährt. Durch die Hydrophilisierung wird der Kapillareffekt ermöglicht bzw. unterstützt.

Die Folie wird mit einer Prägestruktur einer Prägevorrichtung zeitweise derart zusammengefügt, dass Teile der Prägestruktur wenigstens teilweise in die Folie eindringen und eine flächige Matrixstruktur mit einer Mehrzahl von Zellen erzeugen, die an der Oberseite und der Unterseite der Folie Zellöffnungen aufweisen. Die Prägestruktur kann z. B. ein Prägeelement oder ein Prägestempel sein. In einem weiteren Schritt werden die Folie und die Prägestruktur voneinander getrennt, wobei in der Regel die Prägestruktur bewegt wird. In der Folie entsteht eine Mehrzahl von Zellen mit je einem Zellhohlraum, die durch Flüssigkeitspassagen miteinander verbunden sind, so dass eine "Profilfolien-Zellmatrix-Spreitschicht" gebildet wird.

Die Folie kann zusätzlich mittels eines Lasers bearbeitet werden, um Zellöffnungen der Zellen in der Folienseite zu vergrößern oder anzupassen, die der Prägestruktur abgewandt ist. Durch das Laserschneiden lassen sich Öffnungen oder Löcher mit einer Größe von ca. 20 µm oder mehr herstellen. Die eingeprägten oder eingestanzten Zellen können mittels Lasers entgratet werden. Das Laserschneiden ist ein im Stand der Technik gängiges, sehr kostengünstiges Verfahren.

In einer anderen Ausführungsform des automatisierten Verfahrens wird eine zweite Prägestruktur mit der Folie derart zusammen gebracht, dass die Folie zwischen der ersten und der zweiten Prägestruktur angeordnet ist und beide Prägestrukturen wenigstens teilweise in die Folie eindringen. So entstehen Zellen mit Zellöffnungen an den beiden gegenüberliegenden Seiten der Folie, die die Flüssigkeitseintrittsseite und die gegenüberliegende Flüssigkeitsaustrittsseite sind. Die Zellöffnungen können durch eine unterschiedliche Gestaltung der Prägestrukturen verschieden sein, beispielsweise können Größe oder Form voneinander abweichen.

Die Prägestrukturen können beispielsweise als Prägeelemente an Rädern, z.B. Stahlrädern, befestigt sein oder direkt in Prägeräder eingearbeitet sein. Die Prägeräder erlauben eine automatisierte Herstellung. Die Prägestrukturen aus Silizium lassen sich durch Lithographieprozesse und durch Ätzen, Verchromen und erneutes Ätzen technisch einfach herstellen. Silizium ist für die Herstellung der Prägestrukturen sehr gut geeignet. Es ist hart und abriebfest, wegen seiner Brüchigkeit kann man zweckmäßigerweise auch eine Vielzahl von kleineren Elementen verwenden, die zum Beispiel auf eine Prägewälze entsprechend angebracht sind.

Es hat sich als vorteilhaft erwiesen, wenn das Prägen bei Temperaturen erfolgt, die höher als die Raumtemperatur, wenigstens 50 oder 60 Grad, sind. In einigen Fällen können die Temperaturen größer 70 Grad sein. Temperaturen über 90 Grad werden jedoch vermieden.

Die Erfindung wird nachfolgend anhand von in den Figuren dargestellten bevorzugten Ausführungsformen näher erläutert. Die dort dargestellten Besonderheiten können einzeln oder in Kombination verwendet werden, um bevorzugte Ausgestaltungen der Erfindung zu schaffen. Die hier beschriebenen Ausführungen stellen keine Einschränkung der durch die Ansprüche in ihrer Allgemeinheit definierten Erfindung dar. Es zeigen:
- Figur 1: ein erfindungsgemäßes Testelement mit einer Spreitschicht und einem Testfeld;
- Figur 2: die Spreitschicht aus Figur 1;
- Figur 3: ein Steckelement mit dem Testelement aus Figur 1;
- Figur 4: eine alternative Ausführungsform eines Testelements;
- Figur 5a,b: weitere Ausführungsformen einer Spreitschicht;
- Figur 6: eine weitere Ausführung eines Stechelements mit Testelement
- Figur 7: eine weitere Ausführung eines Stechelementes mit Testelement;
- Figur 8: eine weitere Ausführung eines Stechelementes mit Testelement;
- Figur 9: eine Ausführung eines Testelements zur optischen Analyse;
- Figur 10: ein Analysegerät mit dem Testelement gemäß Figur 9;
- Figur 11: eine Prägevorrichtung zur Herstellung einer Spreitschicht.

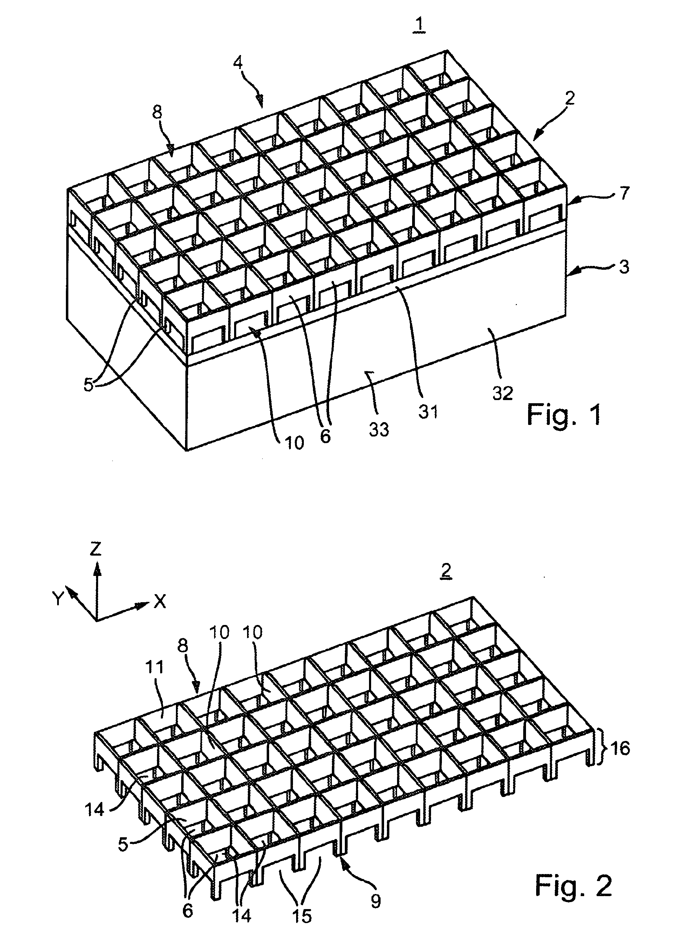

Figur 1 zeigt ein erfindungsgemäßes Testelement 1 mit einer Spreitschicht 2 und einem Testfeld 3, welches mindestens einen Teil der Reagenzien des Reagenzsystems des Testelementes 1 enthält. Die Reaktion des Reagenzsystems mit dem gesuchten Analyten führt zu einer Änderung einer an dem Testelement 1 insbesondere optisch messbaren für das gewünschte analytische Ergebnis charakteristischen Messgröße. Das Testelement 1 ist nicht auf eine optische Messung beschränkt. Die charakteristische Messgröße kann prinzipiell auch nicht-optisch erfasst werden.

Die Spreitschicht 2 hat eine flächige Matrixstruktur 4 mit einer Mehrzahl von Zellen 5, die jeweils einen Zellhohlraum 6 aufweisen. Die Zellen 5 stehen über Flüssigkeitspassagen in Fluidverbindung miteinander. In der gezeigten Ausführungsform (Figur 1, 2) sind alle Zellen 5 der Matrixstruktur 4 in nur einer Ebene 7 angeordnet. Es ist jedoch auch denkbar, dass die Zellen 5 in mehreren sich übereinander erstreckenden Ebenen 7 angeordnet sind.

Die aus einer Folie mittels eines Schichtprofilierungsverfahrens hergestellte Spreitschicht 2 hat eine Flüssigkeitseintrittsseite 8, die parallel zu dem Testelement 3 ausgebildet ist. Eine gegenüberliegende Flüssigkeitsaustrittsseite 9 ist dem Testfeld 3 derart benachbart, dass sie mit einer Flüssigkeitseintrittsseite 10 des Testfelds 3 in Fluidkontakt steht. Die Flüssigkeitseintrittsseite 8 und die Flüssigkeitsaustrittsseite 9 sind im vorliegenden Beispiel gegenüberliegende Seiten der Spreitschicht 2. Selbstverständlich ist es auch möglich, dass eine andere Seite (z.B. eine Schmalseite) der Spreitschicht 2 die Flüssigkeitseintrittsseite ist.

Das Testfeld 3 hat eine Reagenzschicht 31, die der Flüssigkeitsaustrittsseite 9 der Spreitschicht 2 zugewandt ist und die einen Teil der Reagenzien des Reagenzsystems enthält. Eine in der Spreitschicht 2 verteilte Flüssigkeit steht mit der Reagenzschicht 31 derart in Fluidkontakt, dass sie mit den Reagenzien des Reagenzsystems des Testfelds 3 reagieren kann. Die verteilte Flüssigkeit dringt somit gleichmäßig (großflächig) durch die Flüssigkeitseintrittsseite 10 in die Reagenzschicht 31 ein.

Das Testfeld 3 weist eine transparente Tragschicht 32 auf, die von der Spreitschicht 2 abgewandt ist und sich an die Reagenzschicht 31 anschließt. Die bevorzugt flüssigkeitsdichte Tragschicht 32 ist vorzugsweise wenigstens 40 µm dick. In der gezeigten Ausführung ist sie eine ca. 50 µm dicke Folie 34. In der Regel ist die Dicke der Tragschicht 32 nicht größer als 200 µm, bevorzugt nicht größer als 100 µm oder 70 µm.

Figur 2 zeigt die erfindungsgemäße Spreitschicht 2, die als Profilfolien-Zellmatrix-Spreitstruktur ausgebildet und bevorzugt mittels eines Profilierungsverfahrens aus einer Folie hergestellt ist. Die beispielhafte Spreitschicht 2 ist ca. 0,2 mm breit, ca. 0.4 mm lang und hat eine Dicke 16 von 50 µm. Die Zellen 5 der Ebene 7 sind in Reihen und Spalten angeordnet. In der Zellmatrix-Spreitstruktur sind in den Spalten und Reihen jeweils mehrere Zellen angeordnet. Im vorliegenden Beispiel weist jede Spalte fünf Zellen 5 und jede Reihe neun Zellen 5 auf. Vorteilhafterweise sind die einzelnen Zellen so dimensioniert, dass in X-Y-Ebene (an der Flüssigkeitseintrittsseite 8) eine der Dimensionen (Abstand zweier gegenüberliegender Zellwände) höchstens fünf mal so groß ist wie die andere Dimension, bevorzugt höchstens dreimal so groß. Im Gegensatz dazu ist bei den bekannten Kanal-Spreitstrukturen im Stand der Technik der Kanal so dimensioniert, dass er in eine Richtung der X-Y-Ebene deutlich größer ist, in der Regel mehr als 10 mal so groß.

Die Zellen 5 haben einen quadratischen Querschnitt. Selbstverständlich sind auch rechteckige oder runde, z. B. elliptische Querschnitte möglich. Zellwände 10 begrenzen die Zellen 5 in der Ebene 7, wobei die Zellwände 10 deutlich schmäler sind als die Zellhohlräume 6 der Zellen 5.

In besonderen Ausführungsformen könnte die rechteckige Spreitschicht 2 gemäß Figur 2 auch Zellen 5 unterschiedlicher Größe aufweisen. Da die Spreitschicht 2 durch ein Schichtprofilierungsverfahren hergestellt werden kann, können die Zellgrößen nahezu beliebig gestaltet werden, um die Spreitschicht an die jeweilige Anforderung anzupassen. Beispielsweise können die Zellen 5 in den Randbereichen der Spreitschicht 2 andere Größen aufweisen, als die Zellen 5 in der Mitte der Flüssigkeitseintrittsseite 8. Die Zellen 5 in der Mitte der Flüssigkeitseintrittsseite 8 können einen quadratischen Querschnitt aufweisen, während die Zellen 5 in den äußeren Reihen und Spalten jeweils einen rechteckigen Querschnitt haben können, dessen Ausrichtung mit der Ausrichtung der Spreitschicht 2 korrespondiert. Durch die sehr flexible Anpassung der Spreitschicht 2 an die jeweiligen Anforderungen können Spreitschichten mit einer unterschiedlichen Steifigkeit erzielt werden.

Jede der Zellen 5 gemäß Figur 2 hat an der Flüssigkeitseintrittsseite 8 eine Zellöffnung 11, deren Querschnittsdimensionen deutlich größer als die Breite der Zellwände 10 sind. Insgesamt ergibt sich damit eine Gesamtöffnungsfläche aller Zellöffnungen 11, die größer als 50 % der Gesamtfläche der Flüssigkeitseintrittsseite 8 ist. Die Zellöffnungen 11 sind Flüssigkeitseintrittsöffnungen 12, durch Flüssigkeit in die Spreitschicht 2 eintritt. Sie sind so dimensioniert, dass ihre größte Dimension höchstens 75 µm, bevorzugt höchstens 40 µm ist. Besonders bevorzugt ist die größte Dimension der Zellöffnung 20 µm.

Die Zellen 5 haben bevorzugt eine Zellöffnung 11 (Flüssigkeitseintrittsöffnung 12) an der Flüssigkeitseintrittsseite 8 der Spreitschicht 2 und eine Zellöffnung 13 an der Flüssigkeitsaustrittsseite 9. Die Zellöffnungen 11 an der Flüssigkeitseintrittsseite 8 sind kleiner als die Zellöffnungen 13 der Flüssigkeitsaustrittsseite 9, so dass ein Verdunsten der Probenflüssigkeit über die offenen (nicht durch ein Testfeld 3 abgedeckten) Zellöffnungen 11 verhindert wird. Gerade bei kleinen Probenvolumina (und bei längeren Messzeiten) kann die Messung durch verdunstende Flüssigkeit beeinflusst werden.

Zwischen den einzelnen Zellen 5 sind Flüssigkeitspassagen 14 ausgebildet, die die Zellen 5 miteinander verbinden und eine Spreitung und Verteilung der Flüssigkeit auf mehrere Zellen 5 ermöglichen. An den äußeren Zellen 5 der Spreitschicht 2 bilden die Flüssigkeitspassagen 14 Seitenöffnungen 15.

Allgemein ist die Spreitschicht 2 so ausgebildet, so dass eine Mindestschichtdicke einer Flüssigkeitsschicht gewährleistet ist, die an das Testfeld 3 angrenzt. Die Mindestschichtdicke ist so bemessen, dass bei der praktischen Anwendung des Testelements 1 auftretende Variationen der Dicke der Flüssigkeitsschicht nicht zu Änderungen des analytischen Ergebnisses bei unterschiedlichen Messungen führt, die die gewünschte Messgenauigkeit beeinflussen. Die Dicke der Flüssigkeitsschicht und das damit im Zusammenhang stehende Flüssigkeitsvolumen in der Spreitschicht ist kleiner als das Volumen, das durch den Flüssigkeitskanal angeboten wird und für eine Messung zur Verfügung steht. Damit wird erreicht, dass die Spreitschicht stets hinreichend gefüllt ist und das Messergebnis von der primär zur Verfügung stehenden Flüssigkeitsmenge unabhängig ist.

Für die aussagekräftige optische Auswertung der zu untersuchenden Körperflüssigkeit muss die Matrixstruktur 4 der Spreitschicht 2 wenigstens 20, bevorzugt wenigstens 25 Zellen 5 in einer Ebene 7 aufweisen, beispielsweise 5 mal 9, also 35 Zellen 5. In manchen Fällen können 50 Zellen 5 in der Ebene 7 vorteilhaft sein, in anderen Fällen mindestens 100 Zellen 5.

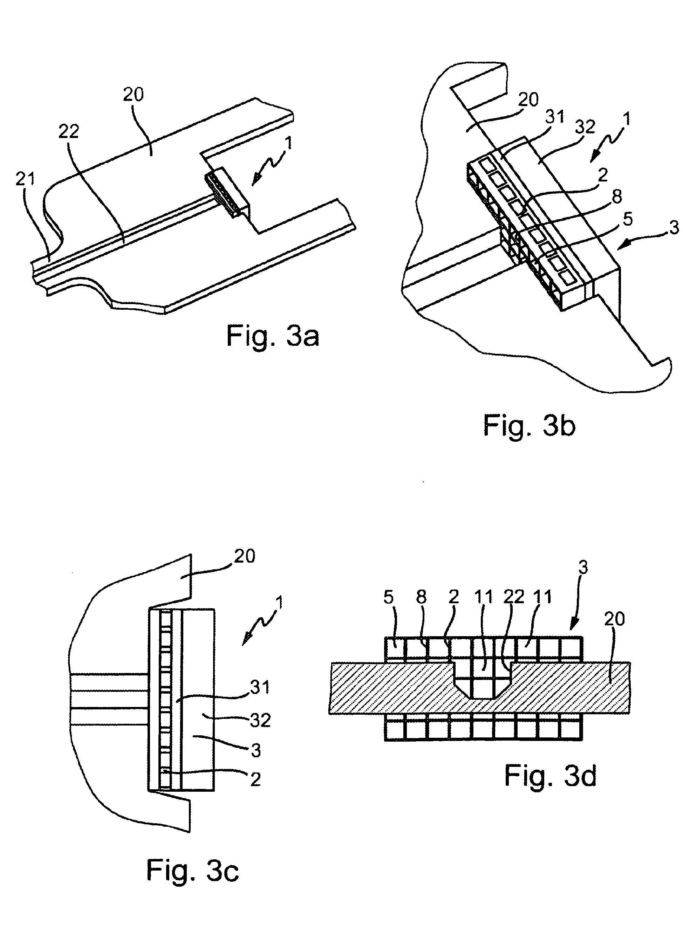

Figur 3 zeigt den Einsatz eines Testelements 1 in Kombination mit einem Stechelement 20. Ein flaches Stechelement 20, wie es für die Blutanalyse und die Glukosebestimmung eingesetzt wird, hat ein Nadelelement 21, an dessen freien Ende eine Spitze zur Erzeugung einer Einstichwunde angeordnet ist. Ein Kapillarkanal 22 erstreckt sich von der Spitze weg zu einem offenen Ende, an das die Spreitschicht 2 des Testelements 1 angrenzt, Figur 3b. Eine durch den Kapillarkanal 22 strömende Analyseflüssigkeit dringt durch einen Teil der Zellen 5 der Flüssigkeitseintrittsseite 8 in die Spreitschicht 2 ein und wird innerhalb der Spreitschicht 2 auf eine größere Anzahl von Zellen 5 verteilt, idealerweise auf alle Zellen 5. Die Verteilung der Flüssigkeit geschieht derart, dass sich eine Flüssigkeitsschicht in einer Mehrzahl von Zellen 5 ausbildet, deren Dicke mindestens 10 µm ist und die an das Testfeld 3 angrenzt, so dass das Testelement 1 in Fluidkontakt mit der Flüssigkeit steht.

Figur 3c zeigt einen Schnitt durch das Testelement 1. An die Flüssigkeitsaustrittsseite 9 der Spreitschicht 2 grenzt die Reagenzschicht 31 des Testfeldes 3 an. Zur optischen Auswertung der Flüssigkeit wird Licht durch die Tragschicht 32 auf die Reagenzschicht 31 geleitet. Das diffus reflektierte Licht wird an der Außenseite 33 der Tragschicht 32 erfasst.

Figur 3d zeigt einen Querschnitt durch den Kapillarkanal 22 des Stechelements 20. Eine Probenflüssigkeit dringt durch die Zellöffnungen 11 in die Spreitschicht 2 ein und wird auf eine Mehrzahl der Zellen 5 verteilt, in dem die Flüssigkeit durch die Flüssigkeitspassagen 14 zwischen den Zellen 5 weiterströmt. Die Verteilung der Flüssigkeit innerhalb der Spreitschicht 2 erfolgt bevorzugt durch Kapillareffekte, ebenso der Transport der Flüssigkeit aus dem Kapillarkanal 22 in die Zellöffnungen 11.

Bevorzugt ist die Spreitschicht 2 der Testelemente 1 so ausgebildet ist, dass eine Mindestschichtdicke einer an das Testfeld 3 angrenzenden Flüssigkeitsschicht gewährleistet ist, die so bemessen ist, dass in der Praxis auftretende Variationen der Dicke der Flüssigkeitsschicht nicht zu einer die gewünschte Messgenauigkeit kompromittierenden Variation des analytischen Ergebnisses führen. Im Rahmen der Erfindung hat sich eine Flüssigkeitsschichtdicke von mindestens 25 µm als hinreichend erwiesen, damit eine in der Praxis auftretende Variation der Flüssigkeitsschicht von wenigen Zehntel µm bis zu maximal 5 µm keine unakzeptable Messungenauigkeit hervorruft.

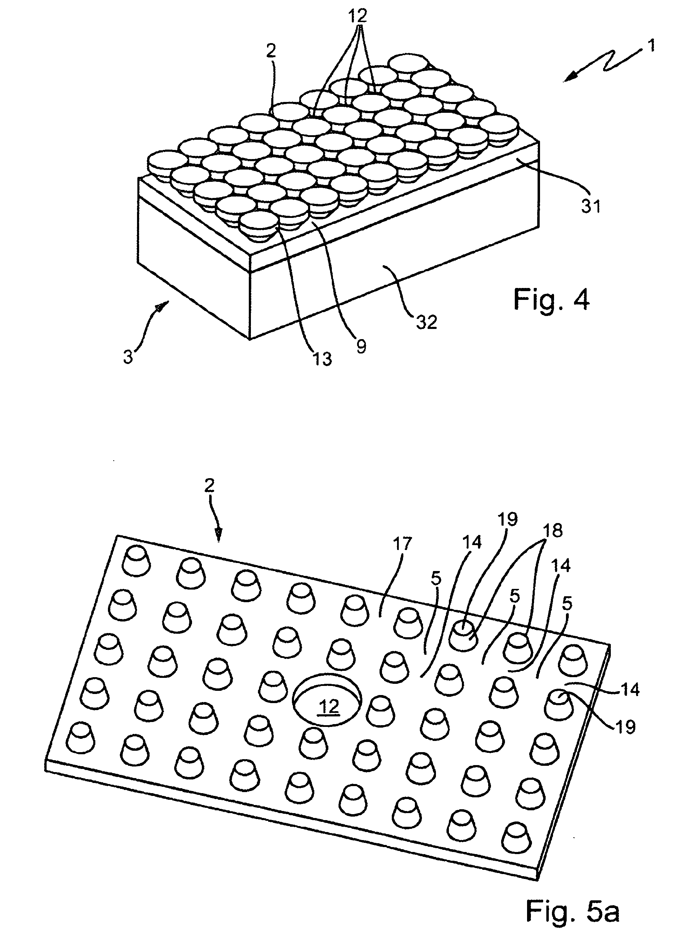

Eine derartige Spreitschicht 2 ist in einer weiteren Ausführungsform eines Testelements 1 gemäß Figur 4 realisiert. Eine noppenartige Spreitschicht 2 hat eine Mehrzahl von Flüssigkeitseintrittsöffnungen 12, die kleiner sind als die Zellöffnungen 13 an der Flüssigkeitsaustrittsseite 9. An die Flüssigkeitsaustrittsseite 9 grenzt die Reagenzschicht 31 des Testfeldes 3 an. Die sich anschließende transparente Tragschicht 32 ist deutlich dicker als die Reagenzschicht 31. In der Praxis weist sie eine Dicke von mehreren 10 µm bis mehreren 100 µm auf. Die Reagenzschicht 31 ist in der Regel mindestens 10 µm, bevorzugt mindestens 20 µm dick, jedoch nicht dicker als 100 µm. Die Reagenzschicht 31 kann aus mehreren Schichten aufgebaut sein und beispielsweise eine Detektionsschicht umfassen, in der eine Reflexion des eintretenden Lichts erfolgt.

Figur 5a zeigt eine weitere Ausführungsform einer Spreitschicht 2 mit einer zentralen Flüssigkeitsöffnung 12 in einer Grundplatte 17. An einer Seite Grundplatte 17 sind eine Vielzahl von Noppen 18 ausgebildet. Je 4 Noppen 18 bilden die Eckpunkte einer Zelle 5. Zwischen den Noppen 18 bestehen Flüssigkeitspassagen 14, die die Zellen 5 miteinander verbinden. Eine durch die Flüssigkeitseintrittsöffnung 12 der Grundplatte 17 eindringende Flüssigkeit wird auf die zwischen den Noppen 18 gebildeten Zellen 5 verteilt. Die Zellen 5 sind nur in einer Ebene ausgebildet. An eine Oberseite 19 der Noppen 18 grenzt die Reagenzschicht 31 eines Testfeldes 3 an, so dass ein Flüssigkeitsübertrag von den Zellen 5 auf das (nicht dargestellte) Testfeld 3 erfolgt.

Die Grundplatte 17 hat eine Größe von 500 µm x 500 µm. Die Noppen 18 haben eine Höhe von 30 µm. Das von der Spreitschicht 2 aufgenommene Flüssigkeitsvolumen ist damit kleiner als 7,5 nl. Die Flüssigkeitsschichtdicke, die von der Spreitschicht 2 zur Verfügung gestellt wird, beträgt maximal 30 µm; in der Regel ist sie etwas kleiner.

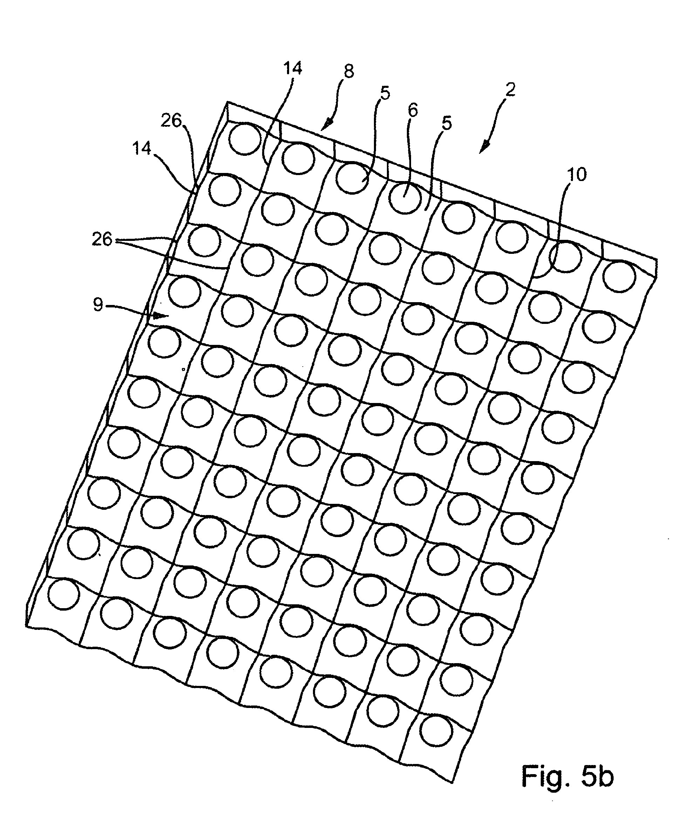

Figur 5b zeigt eine als "Profilfolien-Zellmatrix-Spreitschicht" ausgebildete Spreitschicht 2 mit 80 Zellen 5 (8 Reihen mit je 10 Zellen), bei der die Flüssigkeitseintrittsöffnungen 12 auf der nicht dargestellten Flüssigkeitseintrittsseite 8 rund ausgebildet sind. Bevorzugt erweitern sich die Zellen 5 und ihre Zellhohlräume 6 von der der Flüssigkeitsaustrittsseite 9 gegenüberliegenden Seite zu der Flüssigkeitsaustrittsseite 9 hin. In Figur 5b ist diese Seite die Flüssigkeitseintrittsseite 8. Vorzugsweise erweitern sich die Zellen 5 stetig, wie in Figur 5b gezeigt. Die zwischen den einzelnen Zellen 5 ausgebildeten Zellwände 10 weisen an ihrer der Flüssigkeitsaustrittsseite 9 zugewandten Kante eine Ausnehmung 26 auf, so dass eine Flüssigkeitspassage 14 zwischen zwei benachbarten Zellen 5 gebildet wird.

Die Spreitschicht 2 nach Figur 5b hat eine Größe von 320 µm x 400 µm, die sich aus den 80 Zellen 5 mit quadratischem Querschnitt berechnet, die jeweils eine Seitenkante von 40 µm (an der Flüssigkeitseintrittsseite 8 der Spreitschicht 2) und eine Höhe von 60 µm aufweisen. Jede Zelle 5 hat somit ein Bruttovolumen von 0,096 nl, so dass das Gesamtvolumen der Spreitschicht etwa 7,7 nl beträgt. Da die Spreitschicht 2 ein effektives Flüssigkeitsaufnahmevolumen von ca. 60 % des Gesamtvolumens hat, kann in ihr ein Volumen von ca. 4,5 nl aufgenommen werden. Die mittlere Schichtdicke der Flüssigkeit beträgt ca. 50 µm.

Den bevorzugt mittels eines Profilierungsverfahrens aus einer Folie hergestellten Spreitschichten 2 gemäß den Figuren 2, 4 und 5b ist gemeinsam, dass die Zellöffnungen 11 der Zellen 5 an der Flüssigkeitseintrittsseite 8 kleiner sind als die Zellöffnungen 13 an der Flüssigkeitsaustrittsseite 9. Die kleineren Zellöffnungen 11 verhindern ein Verdunsten der Probenflüssigkeit, was insbesondere bei kleinsten Probenmengen von höchstens 100 nl wichtig ist.

Diese Spreitschichten 2 erfüllen auch die allgemeine Forderung, dass die Gesamtfläche der Zellöffnungen 13 an der Flüssigkeitsaustrittsseite 9 der Spreitschicht 2 wenigstens 75 % der Gesamtfläche der Flüssigkeitsaustrittsseite 9 beträgt. Bevorzugt soll die Gesamtfläche der Zellöffnungen 13 wenigstens 85 %, besonders bevorzugt wenigstens 90 % der Gesamtfläche der Flüssigkeitsaustrittsseite 9 bilden. In den gezeigten Beispielen nach Figur 2 und Figur 5b beträgt die Gesamtfläche der Zellöffnungen 13 ca. 95 % der Gesamtfläche der Flüssigkeitsaustrittsseite 9. Bei einem Gesamtöffnungsgrad aller Zellöffnungen 13 von mehr als 90 %, kann mit einer Spreitschicht 2 mit 20 Zellen 5 eine optische Analyse durchgeführt, bei der der Einfluss der Zellwände 10 zwischen den Zellen 5 ohne Bedeutung für das Analyseergebnis ist. Die Messung kann durch eine statistische Auswertung von 20 Zellen zu einem Ergebnis gelangen, dessen Messgenauigkeit hinreichend groß ist.

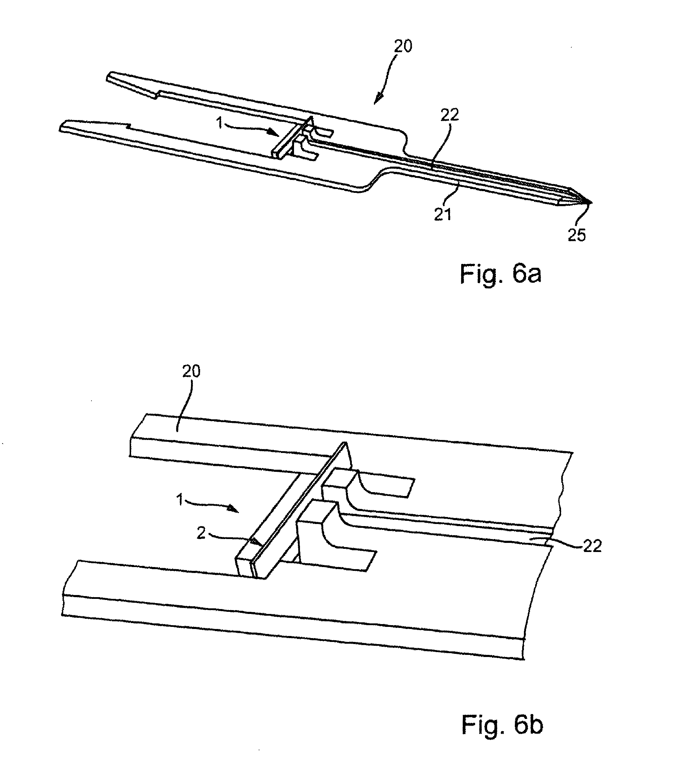

Die Figuren 6a,b zeigen eine weitere Ausführungsform eines Stechelements 20 mit einem Testelement 1. Das flache Stechelement 20 hat ein Nadelelement 21, an dessen freien Ende eine Spitze 25 gebildet ist. Ein halboffener Kapillarkanal 22 erstreckt sich von der Spitze 25 weg. An seinem anderen Ende ist eine Haltestruktur gebildet, an die ein Testelement 1 quer zum Kapillarkanal 22 angeordnet und so gehalten werden kann, dass die Flüssigkeitseintrittsseite 8 der Spreitschicht 2 dem Kapillarkanal 22 benachbart ist. Eine in den Kapillarkanal 22 eindringende Flüssigkeit wird durch Kapillarwirkung in die Spreitschicht 2 transportiert.

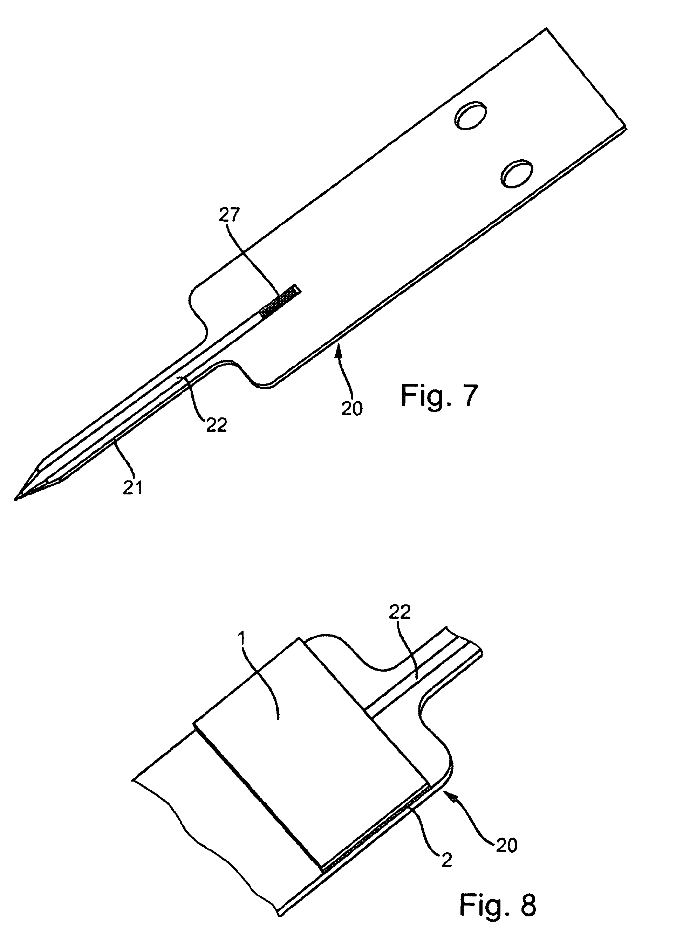

Figur 7 zeigt eine andere Ausführungsform eines flachen Stechelementes 20 mit einem Nadelelement 21. Am Ende des Kapillarkanals 22 ist eine Bohrung 27 vorgesehen, so dass Flüssigkeit aus dem halboffenen Kapillarkanal 22 auf die gegenüberliegende Seite des Stechelementes 20 transportiert werden kann. An der gegenüberliegenden Seite ist ein Testelement 1 so angeordnet, dass die Spreitschicht 2 dem Stechelement 20 benachbart ist und Flüssigkeit von dem Kapillarkanal 22 in die Spreitschicht 2 eindringt. Die Spreitschicht 2 ist durch die Bohrung 27 hindurch zu erkennen.

Eine weitere Ausführungsform eines Stechelementes 20 mit einem halboffenen Kapillarkanal 22 ist in Figur 8 gezeigt. Am Ende des Kapillarkanals 22 überdeckt ein Testelement 1 einen Teil des offenen Kapillarkanals 22. Das Testelement 1 ist so angeordnet, dass die Spreitschicht 2 dem Kapillarkanal 22 und dem Stechelement 20 benachbart ist.

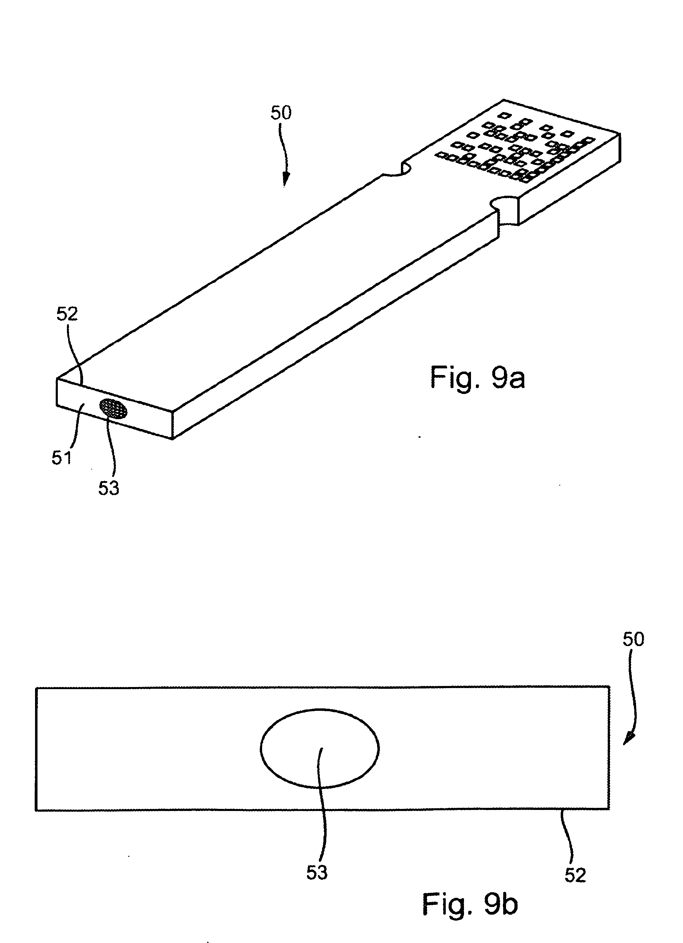

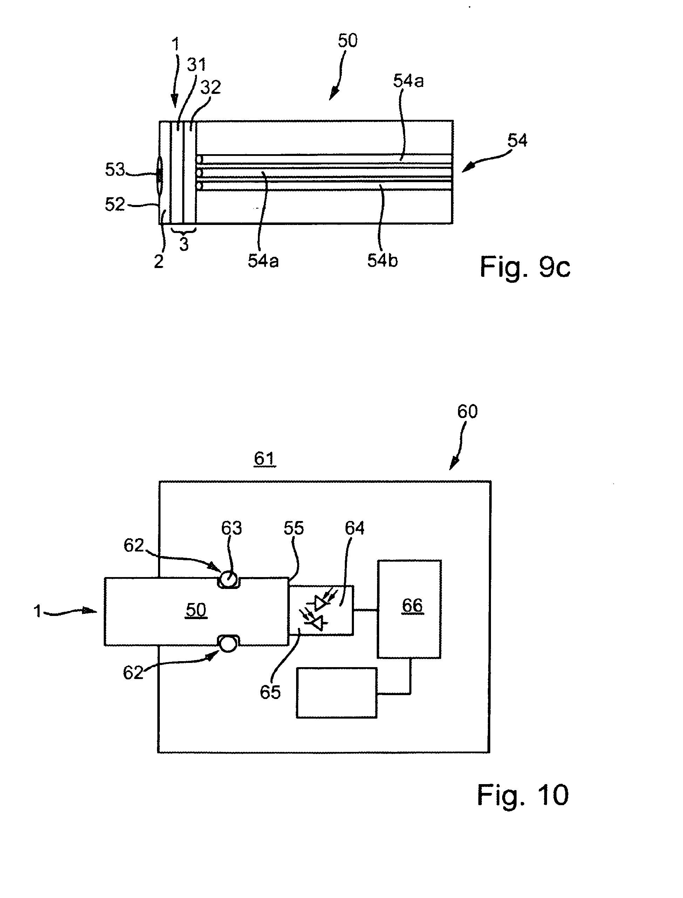

Figur 9 zeigt eine Ausführungsform eines Testelementes 1, das in einen Teststick 50 integriert ist. Der Teststick 50 ist ca. 1 cm breit und ca. 3 bis 5 cm lang, bei einer Höhe von ca. 5 mm. An einem vorderen Ende 51 weist eine Schmalseite 52 des Teststicks 50 eine Öffnung 53 auf, hinter der ein Testelement 1 angeordnet ist. Figur 10b zeigt das vordere Ende 51 des Teststicks 50 im Schnitt. Das Testelement 1 ist in dem Teststick 50 so integriert, dass die Spreitschicht 2 dem vorderen Ende 51 zugewandt und das Testfeld 3 dem vorderen Ende abgewandt ist.

Eine durch die Öffnung 53 an der Schmalseite 52 eindringende Flüssigkeit benetzt eine Mehrzahl von Zellen 5 der Spreitschicht 2 und verteilt die Flüssigkeit auf eine Vielzahl von Zellen 5. Die Spreitschicht 2 kann eine der oben beschriebenen Spreitschichten 2 sein. Eine Flüssigkeit wird derart verteilt und gehalten, dass sich eine Flüssigkeitsschicht mit einer gewünschten Flüssigkeitsdicke bildet, die an die Reagenzschicht 31 des Testfelds 3 angrenzt. Die nun stattfindende Reaktion ist unabhängig von der Flüssigkeitsmenge, solange die Flüssigkeitsschicht in der Spreitschicht 2 eine hinreichende Schichtdicke aufweist, die beispielsweise abhängig von der Messzeit ist, wie oben beschrieben.

An die der Spreitschicht 2 abgewandte Tragschicht 32 des Testfeldes 3 grenzen drei Lichtleiter 54. Einer der Lichtleiter 54, bevorzugt der mittlere Lichtleiter 54a, leitet Licht zu der Tragschicht 32, das durch die Tragschicht 32 hindurch in die Reagenzschicht 31 eindringt und dort diffus reflektiert wird. In Abhängigkeit von der durch die Reaktion mit einer Probenflüssigkeit hervorgerufenen Farbänderung wird das eintretende Licht (stärker oder weniger stark) reflektiert. Das reflektierte Licht wird über wenigstens einen der anderen Lichtleiter 54, bevorzugt über die beiden äußeren Lichtleiter 54b, zu einem optischen Detektor eines Analysengerätes geleitet.

Figur 10 zeigt das Prinzipbild eines Testsystems 61 mit einem Analysegerät 60 und einem Testelement 1. Das Analysegerät 60 hat eine Halterung 62 zur Aufnahme und zum Halten des Testelements 1. Das Testelement 1 ist in den Teststick 50 gemäß Figur 9 integriert. Das Analysegerät 60 ist entsprechend zur Aufnahme des Teststicks 50 mit dem integrierten Testelement 1 ausgebildet. Die Halterung 62 umfasst zwei Haltezylinder 63, die mit Ausnehmungen in den langen Schmalseiten des Teststicks 50 korrespondieren. Die in dem Teststick 50 verlaufenden Lichtleitern 54 leiten das Licht zum hinteren Ende 55 des Teststicks 50.

Das Analysegerät 60 hat einen optischen Detektor 64, der in einer optischen Messeinheit 65 integriert ist. Der Detektor 64 und die optische Messeinheit 65 sind so angeordnet, dass Licht aus der Messeinheit 65 in dem mittleren Lichtleiter 54a des Teststicks 50 geleitet und reflektiertes Licht aus den äußeren Lichtleitern 54b des Teststicks 50 dem Detektor 64 zugeführt werden kann. Mittels einer Auswerte einheit 66 wird das optische Messsignal ausgewertet und ein Analyseergebnis ermittelt. Das Analyseergebnis kann dem Benutzer an einer Anzeigeeinheit 67 angezeigt werden. Aus Gründen der Übersichtlichkeit sind in Figur 10 weitere Komponenten des Analysegeräts 60 nicht dargestellt. Derartige Komponenten können eine Stromversorgungseinheit, ein Mikroprozessor und ähnliche Komponenten, wie z. B. Lautsprecher, sein.

Das Analysegerät 60 kann beispielsweise als Handgerät ausgebildet sein, so dass der Benutzer oder Patient seinen Teststick 50 in das Gerät 60 einlegt oder einclipst und der Blutzuckergehalt innerhalb weniger Sekunden angezeigt wird. Der Blutstropfen zur Analyse wird dazu auf die Öffnung 53 an der vorderen Schmalseite 51 des Teststicks 50 aufgebracht, bevor der Teststick 50 in das Analysegerät 60 eingelegt wird.

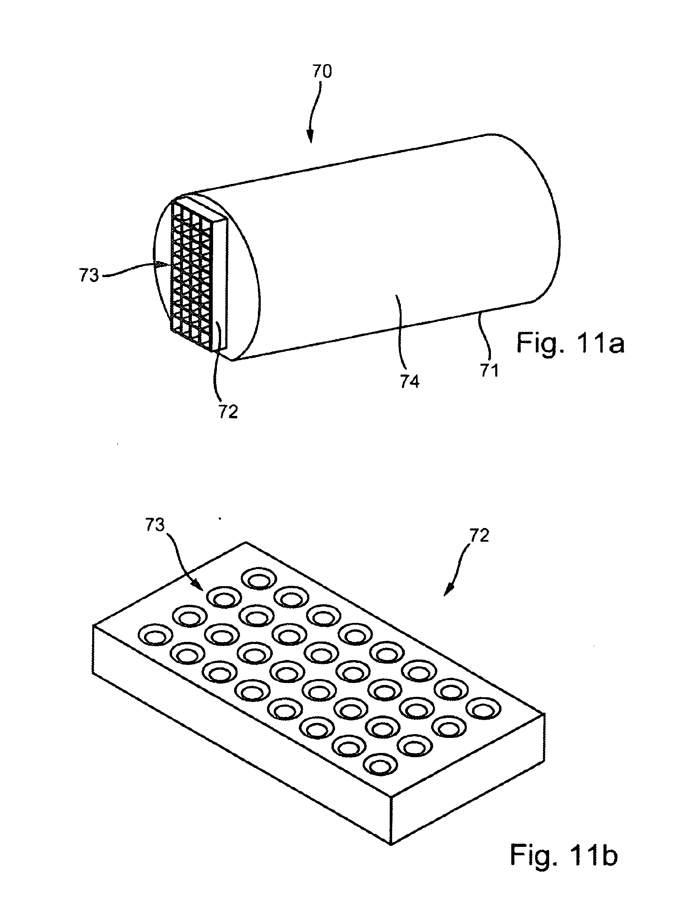

Figur 11a zeigt das Prinzipbild einer als Prägestempel 71 ausgebildeten Prägevorrichtung 70, wobei der Prägestempel 71 einen zylinderartigen Haltekörper 74 und ein Prägeelement 72 umfasst. Das Prägeelement 72 ist an dem Haltekörper 74 auswechselbar befestigt. Das Prägeelement 72 weist eine Prägestruktur 73 auf, die der Zellanordnung und den Zellen der in einer Folie zu erzeugenden Spreitschicht 2 entspricht. Durch einfaches Auswechseln des Prägeelements 72 können Spreitschichten 2 mit unterschiedlicher Struktur und unterschiedlich angeordneten und dimensionierten Zellen 5 auf einfache Weise erzeugt werden. Das Prägeelement 72 in Figur 11b hat beispielsweise eine Prägestruktur 73 mit der eine Spreitschicht 2 erzeugt wird, die eine Zellmatrixstruktur mit 4 x 9 Zellen hat.

## Patentansprüche

1. Testelement zur Analyse einer Körperflüssigkeit auf einen darin enthaltenen Analyten, mittels eines Reagenzsystems, dessen Reaktion mit dem Analyten zu einer Änderung einer an dem Testelement insbesondere optisch messbaren für das gewünschte analytische Ergebnis charakteristischen Messgröße führt, mit
einem Testfeld (3), welches mindestens einen Teil der Reagenzien des Reagenzsystems enthält, und
einer Spreitschicht (2), die derartig parallel zu dem Testfeld (3) verläuft, dass eine Flüssigkeitsaustrittsseite (9) der Spreitschicht (2) in Fluidkontakt zu einer Flüssigkeitseintrittsseite (30) des Testfelds (3) steht und durch die eine in die Spreitschicht (2) eindringende Flüssigkeit so auf deren Flüssigkeitsaustrittsseite (9) verteilt wird, dass sie in die benachbarte Flüssigkeitseintrittsseite (30) des Testfelds (3) eindringt,
wobei
die Spreitschicht (2) eine flächige Matrixstruktur (4) mit einer Mehrzahl von Zellen (5) hat, die je einen Zellhohlraum (6) aufweisen und die Zellhohlräume (6) der Zellen (5) zumindest teilweise durch Flüssigkeitspassagen (14) in Fluidverbindung miteinander stehen,
die Zellen (5) in einer Ebene (7) angeordnet sind und
die Spreitschicht (2) aus einer Folie mittels eines Schichtprofilierungsverfahrens hergestellt ist.

2. Testelement nach Anspruch 1, **dadurch gekennzeichnet, dass** bei mindestens einem Teil der Zellen (5) die Dimension (z) der Zelle (5) senkrecht zu der Ebene (7) verschieden von mindestens einer der Dimensionen (x,y) der Zellen (5) in der Ebene (7) ist.

3. Testelement nach Anspruch 2, **dadurch gekennzeichnet, dass** die Dimension (z) der Zellen (5) senkrecht zu der Ebene (7) größer als die kleinere der Dimensionen (x,y) der Zellen (5) in Richtung der Ebene (7) ist, wobei sie bevorzugt wenigstens 1,5 mal, besonders bevorzugt wenigstens 2 mal so groß ist.

4. Testelement nach einem der vorhergehenden Ansprüche, **dadurch gekennzeichnet, dass** die Spreitschicht (2) nur eine Ebene (7) von Zellen (5) hat.

5. Testelement nach einem der vorhergehenden Ansprüche, **dadurch gekennzeichnet, dass** die Spreitschicht (2) so ausgebildet ist, dass die Dicke einer an das Testfeld (3) angrenzenden Flüssigkeitsschicht mindestens 20 µm, bevorzugt mindestens 30 µm und/oder höchstens 100 µm, bevorzugt höchstens 50 µm, besonders bevorzugt höchstens 40 µm beträgt.

6. Testelement nach einem der vorhergehenden Ansprüche, **dadurch gekennzeichnet, dass** die Matrixstruktur (4) der Spreitschicht (2) wenigstens 20, bevorzugt wenigstens 25 und besonders bevorzugt wenigstens 50 in einer Ebene (7) angeordnete Zellen (5) hat.

7. Testelement nach einem der vorhergehenden Ansprüche, **dadurch gekennzeichnet, dass** sich die Zellen (5) der Spreitschicht (2) von der der Flüssigkeitsaustrittsseite (9) gegenüberliegenden Seite zur Flüssigkeitsaustrittsseite (9) der Spreitschicht (2) erweitern, vorzugsweise stetig erweitern.

8. Testelement nach einem der vorhergehenden Ansprüche, **dadurch gekennzeichnet, dass** mindestens ein Teil der Zellen (5) der Spreitschicht (2) Zellöffnungen (13) an der der Flüssigkeitsaustrittsseite (9) gegenüberliegenden Seite aufweist, durch die die Flüssigkeit in die Spreitschicht (2) eindringt, so dass diese Seite eine Flüssigkeitseintrittsseite (8) bildet, und die größte Dimension dieser Zellöffnungen (11) höchstens 75 µm, bevorzugt höchstens 40 µm, besonders bevorzugt höchstens 20 µm beträgt.

9. Testelement nach Anspruch 8, **dadurch gekennzeichnet, dass** mindestens ein Teil der Zellen (5) sowohl eine Zellöffnung (11) an der Flüssigkeitseintrittsseite (8) der Spreitschicht (2) als auch eine Zellöffnung (13) an der Flüssigkeitsaustrittsseite (9) aufweisen, wobei die Zellöffnungen (11) an der Flüssigkeitseintrittsseite (8) kleiner sind als die Zellöffnungen (13) an der Flüssigkeitsaustrittsseite (9).

10. Testelement nach einem der vorhergehenden Ansprüche, **dadurch gekennzeichnet, dass** die Gesamtfläche der Zellöffnungen (13) an der Flüssigkeitsaustrittsseite (9) wenigstens 75%, bevorzugt wenigstens 85%, besonders bevorzugt wenigstens 90% der Gesamtfläche der Flüssigkeitsaustrittsseite (9) beträgt.

11. Testelement nach einem der vorhergehenden Ansprüche, **dadurch gekennzeichnet, dass** das Testfeld (3) eine der Flüssigkeitsaustrittsseite (9) der Spreitschicht (2) zugewandte, mindestens einen Teil der Reagenzien des Reagenzsystems enthaltenden Reagenzschicht (31) und eine transparente Tragschicht (32) aufweist.

12. Testelement nach Anspruch 11, **dadurch gekennzeichnet, dass** an der Tragschicht (32) des Testfelds (3) ein Lichtleiter (54) so angeordnet ist, dass von dem Testfeld (3) reflektiertes Licht in den Lichtleiter (54) eintritt, um es zu einem optischen Detektor (64) zu leiten.

13. Testelement zur Analyse einer Körperflüssigkeit auf einen darin enthaltenen Analyten mittels eines Reagenzsystems, dessen Reaktion mit dem Analyten zu einer Änderung einer an dem Testelement optisch messbaren für das gewünschte analytische Ergebnis charakteristischen Messgröße führt, mit
einem Testfeld (3), welches mindestens einen Teil der Reagenzien des Reagenzsystems enthält, und
einer Spreitschicht (2), die derartig parallel zu dem Testfeld (3) verläuft, dass eine Flüssigkeitsaustrittsseite (9) der Spreitschicht (2) in Fluidkontakt zu einer Flüssigkeitseintrittsseite (30) des Testfelds (3) steht und durch die eine in die Spreitschicht (2) eindringende Flüssigkeit so auf deren Flüssigkeitsaustrittsseite (9) verteilt wird, dass sie in die benachbarte Flüssigkeitseintrittsseite (30) des Testfelds (3) eindringt,
wobei
die Spreitschicht (2) eine flächige Matrixstruktur (4) mit einer Mehrzahl von Zellen (5) hat, die je einen Zellhohlraum (6) aufweisen und die Zellhohlräume (6) der Zellen (5) zumindest teilweise durch Flüssigkeitspassagen (14) in Fluidverbindung miteinander stehen,
die Zellen (5) in einer Ebene (7) angeordnet sind,
mindestens eine der Dimensionen der Zelle (5) in der Ebene (7) höchstens 40 µm beträgt,
die Dimension (z) der Zelle (5) senkrecht zu der Ebene (7) mindestens 35 µm beträgt,
die Dimension (z) der Zelle (5) senkrecht zu der Ebene (7) größer als die kleinere der Dimensionen (x,y) der Zellen (5) in Richtung der Ebene (7) ist.

14. Testelement nach Anspruch 13, **dadurch gekennzeichnet, dass** die Dimension (z) der Zellen (5) senkrecht zu der Ebene (7) wenigstens 1,5 mal, bevorzugt wenigstens 2 mal so groß ist wie die kleinere der Dimensionen (x,y) der Zellen (5) in Richtung der Ebene (7).

15. Testelement zur Analyse einer Körperflüssigkeit auf einen darin enthaltenen Analyten mittels eines Reagenzsystems, dessen Reaktion mit dem Analyten zu einer Änderung einer an dem Testelement insbesondere optisch messbaren für das gewünschte analytische Ergebnis charakteristischen Messgröße führt, mit
einem Testfeld (3), welches mindestens einen Teil der Reagenzien des Reagenzsystems enthält, und
einer Spreitschicht (2), die derartig parallel zu dem Testfeld (3) verläuft, dass eine Flüssigkeitsaustrittsseite (9) der Spreitschicht (2) in Fluidkontakt zu einer Flüssigkeitseintrittsseite (30) des Testfelds (3) steht und durch die eine in die Spreitschicht (2) eindringende Flüssigkeit so auf deren Flüssigkeitsaustrittsseite (9) verteilt wird, dass sie in die benachbarte Flüssigkeitseintrittsseite (30) des Testfelds (3) eindringt,
wobei
die Spreitschicht (2) eine flächige Matrixstruktur (4) mit einer Mehrzahl von Zellen (5) hat, die je einen Zellhohlraum (6) aufweisen und die Zellhohlräume (6) der Zellen (5) zumindest teilweise durch Flüssigkeitspassagen (14) in Fluidverbindung miteinander stehen,
die Zellen (5) in einer Ebene (7) angeordnet sind,
die Spreitschicht (2) in der Ebene (7) wenigstens einen ersten Bereich mit Zellen (5a) und einen zweiten Bereich mit Zellen (5b) aufweist,
die Größe der Zellen (5a) in dem ersten Bereich verschieden ist von der Größe der Zellen (5b) in dem zweiten Bereich.

16. Testelement nach Anspruch 15, **dadurch gekennzeichnet, dass** die Querschnittsfläche in der X-Y-Ebene (7) der Zellen (5a) in dem ersten Bereich verschieden ist von der Querschnittsfläche in der X-Y-Ebene (7) der Zellen (5b) in dem zweiten Bereich.

17. System zur Analyse einer Körperflüssigkeit auf einen darin enthaltenen Analyten, mit einem Testelement (1) nach einem der vorhergehenden Ansprüche und mit einem Analysengerät (60), das eine Halterung (62) zur Aufnahme des Testelementes (1), einen optischen Detektor (64) und eine Auswerteeinheit (66) zur Auswertung der charakteristischen Messgröße einschließt,
wobei ein in der Halterung (62) des Analysegeräts (60) gehaltenes Testelement (1) derartig positioniert ist, dass von dem Testfeld (3) reflektiertes Licht zu dem optischen Detektor (64) des Analysegeräts (60) geleitet wird.

18. Verfahren zur Herstellung einer Spreitschicht zur homogenen Verteilung einer Flüssigkeitsmenge, insbesondere für ein Testelement (1) nach einem der vorhergehenden Ansprüche, **dadurch gekennzeichnet, dass** das Schichtprofilierungsverfahren zur Herstellung der Spreitschicht (2) folgende Schritte umfasst:
- Zuführen einer Folie mit einer Oberseite, einer Unterseite und einer definierten Dicke von höchstens 100 µm zu einer Prägevorrichtung (70);
- zeitweises Zusammenfügen einer Prägestruktur (73) der Prägevorrichtung (70) und der Folie derart, dass Teile der Prägestruktur (73) wenigstens teilweise in die Folie eindringen und die Spreitschicht (2) mit einer Mehrzahl von Zellen (5) erzeugen, die zumindest teilweise miteinander in Fluidverbindung stehen und an der Oberseite und Unterseite der Folie Zellöffnungen aufweisen;
- Auseinanderbewegen von Prägestruktur (73) und Folie ;
- gegebenenfalls Hydrophilisieren der Spreitschicht (2).

## Claims

1. Test element for the analysis of a body fluid for an analyte contained therein, using a reagent system, whose reaction with the analyte results in a change of a measured value, which is optically measurable on the test element in particular and is characteristic for the desired analytical result, having
a test field (3), which contains at least a part of the reagents of the reagent system, and
a spreading layer (2), which runs parallel to the test field (3) in such a manner that a liquid outlet side (9) of the spreading layer (2) is in fluid contact with a liquid inlet side (30) of the test field (3) and by which a liquid penetrating into the spreading layer (2) is distributed on its liquid outlet side (9) so that it penetrates into the adjacent liquid inlet side (30) of the test field (3),
wherein
the spreading layer (2) has a planar matrix structure (4) having a plurality of cells (5), which each have a cell cavity (6), and the cell cavities (6) of the cells (5) at least partially have a fluid connection to one another through liquid passages (14),
the cells (5) are positioned in one plane (7), and
the spreading layer (2) is produced from a film using a layer profiling method.

2. Test element according to claim 1, **characterized in that**, in at least a part of the cells (5), the dimension (z) of the cell (5) perpendicular to the plane (7) differs from at least one of the dimensions (x,y) of the cells (5) in the plane (7).

3. Test element according to claim 2, **characterized in that** the dimension (z) of the cells (5) perpendicular to the plane (7) is larger than the smaller of the dimensions (x,y) of the cells (5) in the direction of the plane (7), wherein it preferably being at least 1.5 times, particularly preferably at least 2 times as great.

4. Test element according to any one of the preceding claims, **characterized in that** the spreading layer (2) only has one plane (7) of cells (5).

5. Test element according to any one of the preceding claims, **characterized in that** the spreading layer (2) is implemented, so that the thickness of a liquid layer adjoining the test field (3) is at least 20 µm, preferably at least 30 µm and/or at most 100 µm, preferably at most 50 µm, particularly preferably at most 40 µm.

6. Test element according to any one of the preceding claims, **characterized in that** the matrix structure (4) of the spreading layer (2) has at least 20, preferably at least 25, and particularly preferably at least 50 cells (5) positioned in one plane (7).

7. Test element according to any one of the preceding claims, **characterized in that** the cells (5) of the spreading layer (2) widen, preferably widen continuously, from the side opposite to the liquid outlet side (9) toward the liquid outlet side (9) of the spreading layer (2).

8. Test element according to any one of the preceding claims, **characterized in that** at least a part of the cells (5) of the spreading layer (2) have cell openings (13) on the side opposite to the liquid outlet side (9), through which the liquid penetrates into the spreading layer (2), so that this side forms a liquid inlet side (8), and the largest dimension of these cell openings (11) is at most 75 µm, preferably at most 40 µm, particularly preferably at most 20 µm.

9. Test element according to claim 8, **characterized in that** at least a part of the cells (5) have both a cell opening (11) on the liquid inlet side (8) of the spreading layer (2) and also a cell opening (13) on the liquid outlet side (9), the cell openings (11) on the liquid inlet side (8) being lesser than the cell openings (13) on the liquid outlet side (9).

10. Test element according to any one of the preceding claims, **characterized in that** the total area of the cell openings (13) on the liquid outlet side (9) is at least 75%, preferably at least 85%, particularly preferably at least 90% of the total area of the liquid outlet side (9).

11. The test element according to any one of the preceding claims, **characterized in that** the test field (3) has a reagent layer (31), which faces toward the liquid outlet side (9) of the spreading layer (2) and contains at least a part of the reagents of the reagent system, and a transparent support layer (32).

12. Test element according to claim 11, **characterized in that** an optical fiber (54) is positioned on the support layer (32) of the test field (3) so that light reflected from the test field (3) enters the optical fiber (54), in order to conduct it to an optical detector (64).

13. Test element for analyzing a body fluid for an analyte contained therein using a reagent system, whose reaction with the analyte results in a change of a measured value, which is optically measurable on the test element and is characteristic for the desired analytical result, having
a test field (3), which contains at least a part of the reagents of the reagent system, and
a spreading layer (2) which runs parallel to the test field (3) in such a manner that a liquid outlet side (9) of the spreading layer (2) is in fluid contact with a liquid inlet side (30) of the test field (3), by which a liquid penetrating into the spreading layer (2) is distributed on its liquid outlet side (9) so that it penetrates into the adjacent liquid inlet side (30) of the test field (3),
wherein
the spreading layer (2) has a planar matrix structure (4) having a plurality of cells (5), which each have a cell cavity (6), and the cell cavities (6) of the cells (5) are at least partially in fluid connection with one another through liquid passages (14),
the cells (5) are positioned in one plane (7),
at least one of the dimensions of the cell (5) in the plane (7) is at most 40 µm,
the dimension (z) of the cell (5) perpendicular to the plane (7) is at least 35 µm,
the dimension (z) of the cell (5) perpendicular to the plane (7) is larger than the smaller of the dimensions (x,y) of the cells (5) in the direction of the plane (7).

14. Test element according to claim 13, **characterized in that** the dimension (z) of the cells (5) perpendicular to the plane (7) is at least 1.5 times, preferably at least 2 times as larger as the smaller of the dimensions (x,y) of the cells (5) in the direction of the plane (7).

15. Test element for analyzing a body fluid for an analyte contained therein using a reagent system, whose reaction with the analyte results in a change of a measured value, which is particularly optically measurable on the test element and is characteristic for the desired analytical result, having
a test field (3), which contains at least a part of the reagents of the reagent system, and
a spreading layer (2), which runs parallel to the test field (3) in such a manner that a liquid outlet side (9) of the spreading layer (2) is in fluid contact with a liquid inlet side (30) of the test field (3) and by which a liquid penetrating into the spreading layer (2) is distributed on its liquid outlet side (9) so that it penetrates into the adjacent liquid inlet side (30) of the test field (3),
wherein
the spreading layer (2) has a planar matrix structure (4) having a plurality of cells (5), which each have a cell cavity (6), and the cell cavities (6) of the cells (5) at least partially have a fluid connection to one another through liquid passages (14),
the cells (5) are positioned in one plane (7),
the spreading layer (2) in the plane (7) has at least one first area having cells (5a) and one second area having cells (5b),
the size of the cells (5a) in the first area is different from the size of the cells (5b) in the second area.

16. Test element according to claim 15, **characterized in that** the cross-sectional area in the X-Y plane (7) of the cells (5a) in the first area is different from the cross-sectional area in the X-Y plane (7) of the cells (5b) in the second area.

17. System for analyzing a body fluid for an analyte contained therein, having a test element (1) according to any one of the preceding claims and having an analysis device (60), which includes a holder (62) for receiving the test element (1), an optical detector (64), and an evaluation unit (66) for evaluating the characteristic measured value,
wherein a test element (1) held in the holder (62) of the analysis device (60) is positioned in such a manner that light reflected from the test field (3) is conducted to the optical detector (64) of the analysis device (60).

18. Method for producing a spreading layer for the homogeneous distribution of a quantity of liquid, in particular for a test element (1) according to any one of the preceding claims, **characterized in that** the layer profiling method for producing the spreading layer (2) comprises the following steps:
- supplying a film having a top side, a bottom side, and a defined thickness of at most 100 µm to an embossing device (70);
- temporarily joining an embossing structure (73) of the embossing device (70) and the film in such a manner that parts of the embossing structure (73) at least partially penetrate into the film and generate the spreading layer (2) having a plurality of cells (5), which at least partially have a fluid connection with one another and have cell openings on the top side and bottom side of the film;
- moving apart embossing structure (73) and film;
- optionally hydrophilizing the spreading layer (2).

## Revendications

1. Elément de test pour évaluationr un liquide corporel sur un analyte contenu dans celui-ci, au moyen d'un système réactif, dont la réaction avec l'analyte entraîne une variation d'une grandeur de mesure pouvant être mesurée en particulier de manière optique sur l'élément de test et caractéristique du résultat analytique souhaité, comprenant
une zone de test (3), qui contient au moins une partie des réactifs du système réactif, et
une couche d'étalement (2), qui est agencée parallèlement à la zone de test (3), de telle sorte qu'un côté sortie de liquide (9) de la couche d'étalement (2) est en contact fluidique avec un côté entrée de liquide (30) de la zone de test (3) et par laquelle un liquide entrant dans la couche d'étalement (2) est réparti sur son côté sortie de liquide (9), de telle sorte qu'il pénètre dans le côté entrée de liquide (30) voisin de la zone de test (3),
la couche d'étalement (2) ayant une structure de matrice (4) en nappe avec une pluralité de cellules (5), lesquelles présentent chacune une cavité de cellule (6) et les cavités de cellule (6) des cellules (5) étant en liaison fluidique les unes avec les autres au moins en partie par des passages de liquide (14),
les cellules (5) étant disposées dans un plan (7) et
la couche d'étalement (2) étant fabriquée à partir d'un film au moyen d'un procédé de profilage de couche.

2. Elément de test selon la revendication 1, **caractérisé en ce que**, pour au moins une partie des cellules (5), la dimension (z) de la cellule (5), perpendiculairement au plan (7), est différente d'au moins l'une des dimensions (x, y) des cellules (5) dans le plan (7).

3. Elément de test selon la revendication 2, **caractérisé en ce que** la dimension (z) des cellules (5) perpendiculairement au plan (7) est supérieure à la plus petite des dimensions (x, y) des cellules (5) en direction du plan (7), sachant qu'elle est de préférence au moins 1,5 fois, avec une préférence particulière au moins 2 fois plus grande.

4. Elément de test selon l'une quelconque des revendications précédentes, **caractérisé en ce que** la couche d'étalement (2) a seulement un plan (7) de cellules (5).

5. Elément de test selon l'une quelconque des revendications précédentes, **caractérisé en ce que** la couche d'étalement (2) est conçue de telle sorte que l'épaisseur d'une couche de liquide contiguë à la zone de test (3) est d'au moins 20 µm, de préférence d'au moins 30 µm et/ou au maximum de 100 µm, de préférence au maximum de 50 µm, avec une préférence particulière au maximum de 40 µm.

6. Elément de test selon l'une quelconque des revendications précédentes, **caractérisé en ce que** la structure de matrice (4) de la couche d'étalement (2) a au moins 20, de préférence au moins 25 et avec une préférence particulière au moins 50 cellules (5) disposées dans un plan (7).

7. Elément de test selon l'une quelconque des revendications précédentes, **caractérisé en ce que** les cellules (5) de la couche d'étalement (2) s'élargissent, de préférence constamment, depuis le côté opposé au côté sortie de liquide (9) vers le côté sortie de liquide (9) de la couche d'étalement (2).

8. Elément de test selon l'une quelconque des revendications précédentes, **caractérisé en ce qu'**au moins une partie des cellules (5) de la couche d'étalement (2) présente des ouvertures de cellule (13) sur le côté opposé au côté sortie de liquide (9), par lequel le liquide pénètre dans la couche d'étalement (2), de sorte que ce côté forme un côté entrée de liquide (8), et la plus grande dimension de ces ouvertures de cellule (11) est d'au maximum 75 µm, avec une préférence maximale de 40 µm, avec une préférence particulière d'au maximum 20 µm.

9. Elément de test selon la revendication 8, **caractérisé en ce qu'**au moins une partie des cellules (5) présente aussi bien une ouverture de cellule (11) sur le côté entrée de liquide (8) de la couche d'étalement (2) qu'une ouverture de cellule (13) sur le côté sortie de liquide (9), les ouvertures de cellule (11) sur le côté entrée de liquide (8) étant inférieures aux ouvertures de cellule (13) sur le côté sortie de liquide (9).

10. Elément de test selon l'une quelconque des revendications précédentes, **caractérisé en ce que** la surface totale des ouvertures de cellule (13) sur le côté sortie de liquide (9) représente au moins 75 %, de préférence au moins 85 %, avec une préférence particulière au moins 90 % de la surface totale du côté sortie de liquide (9).

11. Elément de test selon l'une quelconque des revendications précédentes, **caractérisé en ce que** la zone de test (3) présente une couche de réactif (31) tournée vers le côté sortie de liquide (9) de la couche d'étalement (2), contenant au moins une partie des réactifs du système réactif, et une couche de support (32) transparente.

12. Elément de test selon la revendication 11, **caractérisé en ce qu'**un guide de lumière (54) est disposé sur la couche de support (32) de la zone de test (3), de telle sorte que de la lumière réfléchie par la zone de test (3) entre dans le guide de lumière (54) pour la guider vers un détecteur (64) optique.

13. Elément de test pour évaluationr un liquide corporel sur un analyte contenu dans celui-ci au moyen d'un système réactif, dont la réaction avec l'analyte entraîne une variation d'une grandeur de mesure pouvant être mesurée de manière optique sur l'élément de test et caractéristique du résultat analytique souhaité, comprenant
une zone de test (3), qui contient au moins une partie des réactifs du système réactif, et
une couche d'étalement (2), qui est agencée parallèlement à la zone de test (3), de telle sorte qu'un côté sortie de liquide (9) de la couche d'étalement (2) est en contact fluidique avec un côté entrée de liquide (30) de la zone de test (3) et par laquelle un liquide pénétrant dans la couche d'étalement (2) est réparti sur son côté sortie de liquide (9), de telle sorte qu'il pénètre dans le côté entrée de liquide (30) voisin de la zone de test (3),
la couche d'étalement (2) ayant une structure matricielle (4) en nappe avec une pluralité de cellules (5), qui présentent chacune une cavité de cellule (6) et les cavités (6) des cellules (5) étant en liaison fluidique les unes avec les autres au moins partiellement par des passages de liquide (14),
les cellules (5) étant disposées dans un plan (7),
au moins l'une des dimensions de la cellule (5) dans le plan (7) étant d'au maximum 40 µm,
la dimension (z) de la cellule (5) étant d'au moins 35 µm perpendiculairement au plan (7),
la dimension (z) de la cellule (5) perpendiculairement au plan (7) étant supérieure à la plus petite des dimensions (x, y) des cellules (5) en direction du plan (7).

14. Elément de test selon la revendication 13, **caractérisé en ce que** la dimension (z) des cellules (5) perpendiculairement au plan (7) est au moins 1,5 fois, de préférence au moins 2 fois plus grande que la plus petite des dimensions (x, y) des cellules (5) en direction du plan (7).

15. Elément de test pour évaluationr un liquide corporel sur un analyte contenu dans celui-ci au moyen d'un système réactif, dont la réaction avec l'analyte entraîne une variation d'une grandeur de mesure pouvant être mesurée en particulier de manière optique sur l'élément de test et caractéristique du résultat analytique souhaité, comprenant
une zone de test (3), qui contient au moins une partie des réactifs du système réactif, et
une couche d'étalement (2), qui est agencée parallèlement à la zone de test (3), de telle sorte qu'un côté sortie de liquide (9) de la couche d'étalement (2) est en contact fluidique avec un côté entrée de liquide (30) de la zone de test (3) et par laquelle un liquide pénétrant dans la couche d'étalement (2) est réparti sur son côté sortie de liquide (9), de telle sorte qu'il pénètre dans le côté entrée de liquide (30) voisin de la zone de test (3),
la couche d'étalement (2) présentant une structure matricielle (4) en nappe avec une pluralité de cellules (5), lesquelles présentent chacune une cavité de cellule (6) et les cavités (6) des cellules (5) étant en liaison fluidique les unes avec les autres au moins partiellement par des passages de liquide (14),
les cellules (5) étant disposées dans un plan (7),
la couche d'étalement (2) dans le plan (7) présentant au moins une première zone avec des cellules (5a) et une seconde zone avec des cellules (5b),
la grandeur des cellules (5a) dans la première zone étant différente de la grandeur des cellules (5b) dans la seconde zone.

16. Elément de test selon la revendication 15, **caractérisé en ce que** la surface de section dans le plan X-Y (7) des cellules (5a) dans la première zone est différente de la surface de section dans le plan X-Y (7) des cellules (5b) dans la seconde zone.

17. Système pour évaluationr un liquide corporel sur un analyte contenu dans celui-ci, comprenant un élément de test (1) selon l'une quelconque des revendications précédentes et comprenant un appareil d'évaluation (60), qui inclut un support (62) pour le logement de l'élément de test (1), un détecteur (64) optique et une unité d'évaluation (66) pour l'évaluation de la grandeur de mesure caractéristique,
un élément de test (1) maintenu dans la fixation (62) de l'appareil d'évaluation (60) étant positionné de telle sorte que de la lumière réfléchie par la zone de test (3) est dirigée vers le détecteur (64) optique de l'appareil d'évaluation (60).

18. Procédé pour fabriquer une couche d'étalement pour la répartition homogène d'une quantité de liquide, en particulier pour un élément de test (1) selon l'une quelconque des revendications précédentes, **caractérisé en ce que** le procédé de profilage de couche pour la fabrication de la couche d'étalement (2) comprend les étapes suivantes :
- amenée d'un film avec un côté supérieur, un côté inférieur et une épaisseur définie d'au maximum 100 µm à un dispositif d'estampage (70) ;
- assemblage temporaire d'une structure d'estampage (73) du dispositif d'estampage (70) et du film, de telle sorte que des parties de la structure d'estampage (73) pénètrent au moins en partie dans le film et génèrent la couche d'étalement (2) avec une pluralité de cellules (5), qui sont en liaison fluidique les unes avec les autres au moins partiellement et présentent des ouvertures de cellule sur le côté supérieur et le côté inférieur du film ;
- éloignement de la structure d'estampage (73) et du film ;
- éventuellement hydrophilisation de la couche d'étalement (2).
